(19) Europäisches Patentamt
European Patent Office
Office européen des brevets

(11) EP 0 721 455 B1

# (12) FASCICULE DE BREVET EUROPEEN

(45) Date de publication et mention de la délivrance du brevet:
**11.12.2002 Bulletin 2002/50**

(21) Numéro de dépôt: **95928478.7**

(22) Date de dépôt: **26.07.1995**

(51) Int Cl.[7]: **C07D 521/00**, C07D 401/06, A61K 31/445

(86) Numéro de dépôt international:
**PCT/EP95/02978**

(87) Numéro de publication internationale:
**WO 96/004287 (15.02.1996 Gazette 1996/08)**

## (54) TETRAHYDROPYRIDINE-(OU 4-HYDROXYPIPERIDINE) ALKYLAZOLES AYANT UNE ACTIVITE POUR LES RECEPTEURS SIGMA ET/OU 5HT1A

TETRAHYDROPYRIDIN-(ODER 4-HYDROXYPIPERIDIN) ALKYLAZOLE MIT SIGMA UND/ODER 5HT 1A-REZEPTOR AKTIVITÄT

TETRAHYDROPYRIDINE-(OR 4-HYDROXYPIPERIDINE)ALKYLAZOLES HAVING AN AFFINITY FOR SIGMA AND/OR 5HT1A RECEPTORS

(84) Etats contractants désignés:
**AT BE CH DE DK FR GB GR IE IT LI LU MC NL PT SE**

(30) Priorité: **29.07.1994 FR 9409443**

(43) Date de publication de la demande:
**17.07.1996 Bulletin 1996/29**

(73) Titulaire: **LABORATORIOS DEL DR. ESTEVE, S.A.**
**08026 Barcelona (ES)**

(72) Inventeurs:
- **MERCE-VIDAL, Ramon**
  **E-08014 Barcelone (ES)**
- **FRIGOLA-CONSTANSA, Jordi**
  **E-08013 Barcelone (ES)**

(74) Mandataire: **Ahner, Francis et al**
**Cabinet Régimbeau**
**20, rue de Chazelles**
**75847 Paris cedex 17 (FR)**

(56) Documents cités:

| | |
|---|---|
| **EP-A- 0 436 157** | **EP-A- 0 441 349** |
| **EP-A- 0 445 701** | **EP-A- 0 497 658** |
| **EP-A- 0 497 659** | **WO-A-94/13659** |
| **WO-A-95/03298** | **BE-A- 890 222** |
| **DE-A- 2 017 265** | **DE-A- 2 632 870** |
| **DE-A- 3 307 395** | **US-A- 3 509 153** |
| **US-A- 3 981 881** | **US-A- 3 994 904** |
| **US-A- 4 539 407** | **US-A- 4 675 403** |
| **US-A- 4 754 038** | **US-A- 5 296 487** |
| **US-A- 5 411 960** | |

- **J. PHARMACOL. EXPTL. THER., vol.148, no.1, 1965 pages 54 - 65 R. RODRIGUEZ ET. AL. 'Pharmacology of a Group of Phenylpiperazine Tetrazole Derivatives with Adrenergic Blocking Actions'**
- **PATENT ABSTRACTS OF JAPAN vol. 17, no. 135 (C-1037) (5764) 19 Mars 1993 & JP,A,04 308 569 (SUMITOMO) 30 Octobre 1992**
- **PATENT ABSTRACTS OF JAPAN vol. 7, no. 268 (C-197) (1413) 30 Novembre 1983 & JP,A,58 150 511 (EISAI) 7 Septembre 1983**
- **BIOORG. MED. CHEM. LETT., vol.4, no.18, 1994 pages 2185 - 2188 R. L. HUDKINS 'Phenytoin Derivatives as Potent .sigma.-Ligands'**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 721 455 B1

Printed by Jouve, 75001 PARIS (FR)

## Description

La présente invention concerne de nouveaux 4-aryltétrahydropyridines et 4-arylpipéridinols unis à des alkylazoles de formule générale (I)

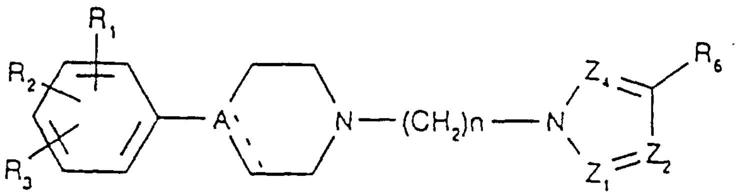

(I)

et leurs sels physiologiquement acceptables, les procédés pour leur préparation, leur application comme médicaments et les compositions pharmaceutiques qui les contiennent.

Les composés objets de l'invention peuvent être également utilisés dans l'industrie pharmaceutique comme intermédiaires et pour la préparation de médicaments.

Ces composés possèdent une puissante affinité pour les récepteurs sigma et/ou 5HT1A et sont donc potentiellement utiles dans le traitement de certains désordres psychiques et neurologiques des êtres humains et autres mammifères.

Il existe des faits qui impliquent les récepteurs sigma dans le traitement de la psychose. De nombreux antipsychotiques atypiques comme le rimcazole (Schwarcz, G. et al., Drug Dev. Res., 1985, 5, 387), le remoxipride (Wadworth, A.N. et al., Drugs 1990, 40, 863) la thiospirone (Jain, A.K. et al, Int. Clin. Psychopharmacol. 1987, 2, 129) montrent une affinité significative pour les récepteurs sigma.

D'autre part, des études de la biologie et de la fonction des récepteurs sigma indiquent que les coordinats du récepteur sigma peuvent être efficaces dans le traitement de certains désordres moteurs, en particulier la chorée de Huntington, la dystonie et le syndrome de Tourette. La présence de récepteurs sigma dans la substance noire permet leur utilisation dans le traitement de la maladie de Parkinson (Walker, J.M. et coll. Pharmacological Reviews, 1990, 42, 355).

Certains coordinats des récepteurs sigma sont en relation avec la modulation des effets produits par la médiation du récepteur NMDA et agissent comme agents anti-ischémiques dans des tests in vivo (Rao, T.S. et al., Molecular Pharmacology, 1990, 37, 978), avec possibilité d'utilisation comme neuroprotecteurs et dans le traitement de l'épilepsie et de la convulsion (Kaiser C. Neurotransmissions VII, 1991).

On a dit que les coordinats des récepteurs sigma présentent des effets anti-amnésiques dans des modèles animaux (Early et al., Brain Research 1991, 546, 281).

Les coordinats sigma influent sur les niveaux d'acétylcholine dans des modèles animaux (Matsuno et al., Brain Research 1992, 575, 315) et peuvent par conséquent être utilisés dans le traitement de la démence sénile, par exemple de type Alzheimer.

Les coordinats des récepteurs 5-HT1A, en particulier agonistes ou agonistes partiels de 5-HT1A, montrent une activité anxiolytique et antidépressive prouvée (Glitz, D.A. Drugs, 1991, 41, 11).

Par conséquent, les agents ayant une puissante affinité pour les récepteurs sigma et/ou 5-HT1A peuvent être utilisés dans un ou plusieurs des traitements indiqués.

Dans la bibliographie on trouve des exemples de 4-aryl-1,2,3,6-tétrahydropyridines et de 4-aryl-4-hydroxypipéridines ; toutefois, on ne trouve pas de description de composés dans lesquels ces sous-structures sont réunies à l'azote d'un noyau azole au moyen d'une chaîne alkyle non substituée :

Davis L. Temple et al., US Patent 4,320,131; 16 mars 1982
Richard A. Glennon et al., J. Med. Chem., 1991, 34, 3360-65
Jean-Luc Malleron et al., J. Med. Chem. 1991, 34, 8, 2477-83
Henning Böttcher et al., J. Med. Chem., 1992, 35, 4020-26
Zhuihua Sui et al., Synthesis, 1993, 803-8
David I. Schuster et al., J. Med. Chem., 1993, 36, 3923-28
David J. Wustrow et al., BioMed. Chem. Lett., 1993, 3, 277-80
Shimazaki Norihiko et al., Can. Pat. App., CA 2053475 AA.

Les inventeurs ont décrit antérieurement une série de N-alkylazoles réunis à l'azote de divers hétérocycles, utiles comme agents non benzodiazépiniques pour le traitement de l'anxiété (brevets européens N° EP 382637, EP 497659 et EP 502786) et pour le traitement d'autres désordres du comportement (brevets européens EP 429360 et EP 497658). Dans les brevets cités sont décrits des composés de formule générale (I), dans laquelle A représente dans tous les cas un atome d'azote, et il s'agit par conséquent d'un noyau pipérazine. Dans la présente invention on remplace le noyau pipérazine par une pipéridine ou une tétrahydropyridine.

Les composés objets de l'invention répondent à la formule générale (I)

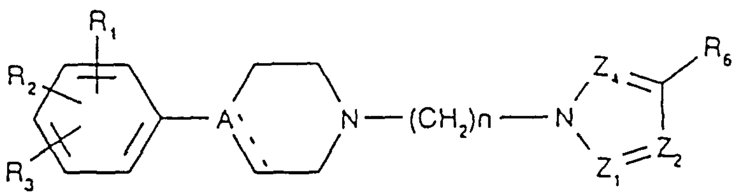

(I)

dans laquelle

$R_1$, $R_2$ et $R_3$, identiques ou différents, représentent chacun un atome d'hydrogène, un atome d'halogène, un radical alkyle linéaire ou ramifié, un radical perhalogénoalkyle, un radical aryle ou aryle substitué ou un radical alcoxyle. De plus, deux radicaux adjacents peuvent former un cycle aromatique ou saturé.
A représente un atome de carbone et la ligne pointillée représente une liaison additionnelle, ou bien A représente un atome de carbone uni à un groupe hydroxyle (C-OH) et la ligne pointillée représente un défaut de liaison additionnelle.
n peut avoir des valeurs allant de 2 à 6, de préférence 4,
$Z_1$ représente un atome d'azote ou un atome de carbone substitué qui peut être représenté par C-$R_4$
$Z_2$ représente un atome d'azote ou un atome de carbone substitué qui peut être représenté par C-$R_5$
$Z_4$ représente un atome d'azote ou un atome de carbone substitué qui peut être représenté par C-$R_7$
$R_4$, $R_5$, $R_6$ et $R_7$, identiques ou différents, représentent un atome d'hydrogène, un atome d'halogène, un radical alkyle linéaire ou ramifié, un radical hydroxyle, un radical alcoxyle, un radical carboxylique, un

Le document US 4 675 403 décrit par ailleurs des composés 4-hydroxy-pipéridine-alkyl-imidazolidine présentant une activité antipsychotique et anxiolytique. Ces composés comportent notamment un hétérocycle saturé azoté substitué par deux groupements carbonyles.

La demande de brevet WO 94/136 59 a pour objet des imidazolidinones, des imidazolones, des benzimidazolines et des pipérazines comprenant des fonctions carbonyles ayant une affinité pour les récepteurs 5-$HT_{1A}$.

Des dérivés de la tétrahydropyridine ayant une affinité pour les récepteurs $\gamma$ sont décrits dans la demande de brevet EP 445 701. Ces dérivés sont substitués par un hétérocycle azoté saturé, une amine ou un amide. Ils peuvent être utilisés comme agent anti-pyschotique.

Hudkins et al. décrivent dans Biorganic and medicinal chemistry letters, vol.4, n°18, pages 2185-2188, 1994, des ligonols des récepteurs $\gamma$ dont la structure de base correspond à des 5,5-diphénylhydantoires substitués par un 4-phénylpipéridinyle ou un 4-phénylpipérazinyle. radical carboxamide, un radical carboxylate d'alkyle, un radical aryle ou aryle substitué, ou bien deux radicaux adjacents peuvent faire partie d'un autre cycle, aromatique ou non.

L'invention concerne également les sels physiologiquement acceptables des composés de formule générale (1), en particulier les sels des acides chlorhydrique, bromhydrique, sulfurique, phosphorique, acétique, lactique, malonique, succinique, glutarique, fumarique, malique, tartrique, citrique, ascorbique, maléique, benzoïque, phénylacétique, cinnamique, salicylique, et des acides alkyl-, cycloalkyl- ou arylsulfoniques.

Le radical

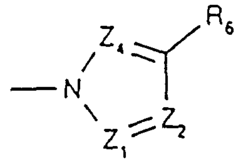

représente un azole, à l'exception des tétrazoles pour lesquels $Z_1$, $Z_2$ et $Z_4$ représentent simultanément un atome d'azote.

Lorsque $R_4$, $R_5$, $R_6$ et $R_7$ ne font pas partie d'un autre cycle aromatique ou non, il s'agit de préférence d'un azole pour lequel un seul de $Z_1$, $Z_2$ ou $Z_4$ représente un atome d'azote, en particulier un pyrazole ($Z_1$ ou $Z_4$ = N) ou un imidazole ($Z_2$ = N).

Lorsque $R_4$, $R_5$, $R_6$ ou $R_7$ font partie d'un autre cycle aromatique ou non, la composante "azole" du radical ci-dessus reste essentielle, c'est-à-dire qu'il n'englobe pas des radicaux tels que des β ou γ carbolines, les pyrroloazépines, et des radicaux comprenant des fonctions carbonyles telles que les pyrazolones, imidazolones, benzimidazolones et d'une manière générale les hétérocycles comprenant une fonction carbonyle.

D'une manière préférentielle, lorsque $R_4$, $R_5$, $R_6$ ou $R_7$ font partie d'un autre cycle aromatique ou non, le radical ci-dessus représente de préférence un radical bicyclique, en particulier un indole, un indazole, un benzimidazole ou un benzotriazole.

Par alkyle, linéaire ou ramifié, on entend de préférence un alkyle inférieur en $C_1$-$C_6$, en particulier un méthyle, un éthyle ou un propyle, cette définition s'appliquant également aux restes alkyles des radicaux alcoxy et carboxylate d'alkyle.

Par aryle ou aryle substitué, on entend de préférence un phényle, éventuellement substitué par un ou plusieurs radicaux sélectionnés parmi les halogènes et les alkyles linéaires ou ramifiés.

Par halogène, on entend le fluor, le chlore ou le brome, de préférence le chlore.

Enfin, par perhalogénoalkyle, on entend un radical alkyle linéaire ou ramifié dont la totalité des atomes d'hydrogène est remplacée par des halogènes. Il s'agit de préférence d'un radical perfluoroalkyle, en particulier le trifluorométhyle.

On peut préparer les nouveaux dérivés de formule générale (I) selon les procédés suivants :

Procédé A :

Par réaction d'un dérivé spiranne de formule générale (II)

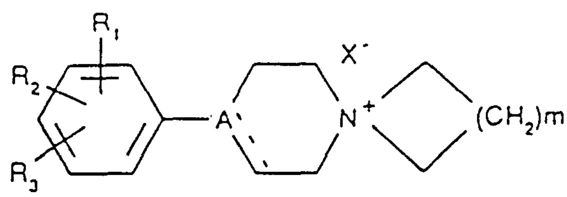

(II)

dans laquelle

$R_1$, $R_2$, $R_3$ et A ont la signification indiquée ci-dessus, m peut avoir des valeurs allant de 0 à 4, et X représente un groupe sortant, comme chloro, bromo, mésyloxy ou tosyloxy,

avec un hétérocycle azoté de formule générale (III)

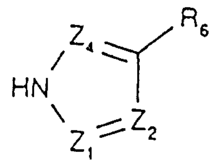

( III )

dans laquelle

$Z_1$, $Z_2$, $Z_4$ et $R_6$ ont la signification indiquée ci-dessus.

La réaction s'effectue dans une solution de diméthylsulfoxyde, diméthylformamide, un alcool comme l'éthanol, un hydrocarbure aromatique comme le toluène ou un hydrocarbure aliphatique comme l'hexane ou un éther comme le dioxanne. Cette réaction est conduite de préférence en présence d'une base comme le carbonate de potassium ou la triéthylamine.

La température de réaction varie entre la température ambiante et la température de reflux du solvant utilisé.

Les temps de réaction varient entre 1 et 24 heures.

<u>Procédé B</u> :

Par réaction simultanée "one pot" entre un dérivé de formule générale (IV), un agent alkylant de formule générale (V) et un hétérocycle azoté de formule générale (III).

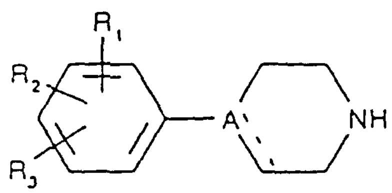

( IV )

$X-(CH_2)n-X$

( V )

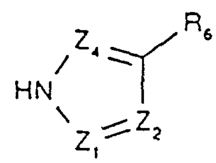

( III )

où

$R_1$, $R_2$, $R_3$, A, X, n, $Z_1$, $Z_2$, $Z_4$ et $R_6$ ont la signification donnée ci-dessus.

La réaction est conduite dans une solution de diméthylsulfoxyde, diméthylformamide, un alcool comme l'éthanol, un hydrocarbure aromatique comme le toluène ou un hydrocarbure aliphatique comme l'hexane ou un éther comme le dioxanne. Cette réaction est conduite de préférence en présence d'une base comme le carbonate de potassium ou la triéthylamine.

La température de réaction varie entre la température ambiante et la température de reflux du solvant utilisé.

Les temps de réaction varient entre 1 et 24 heures.

<u>Procédé C</u>:

La préparation des composés de formule général (1) peut s'effectuer par réaction dans des conditions d'alkylation des amines de formule générale (IV)

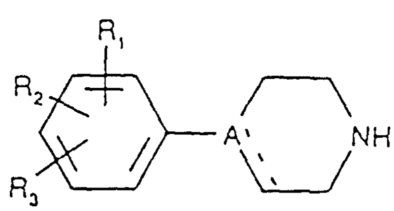

( IV )

dans laquelle
$R_1$, $R_2$, $R_3$ et A ont la signification donnée ci-dessus, avec les composés de formule générale (VI)

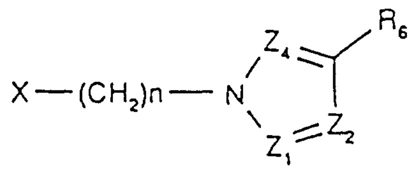

( VI )

dans laquelle
X, n, $Z_1$, $Z_2$, $Z_4$ et $R_6$ ont la signification indiquée ci-dessus.

La réaction est conduite dans une solution de diméthylsulfoxyde, diméthylformamide, un alcool comme l'éthanol, un hydrocarbure aromatique comme le toluène ou un hydrocarbure aliphatique comme l'hexane ou un éther comme le dioxanne. Cette réaction s'effectue de préférence en présence d'une base comme le carbonate de potassium ou la triéthylamine.

La température de réaction varie entre la température ambiante et la température de reflux du solvant utilisé.

Les temps de réaction varient entre 1 et 24 heures.

Procédé D :

On peut procéder à la préparation des composés de formule générale (I) par réaction dans des conditions d'alkylation d'un composé de formule générale (VII)

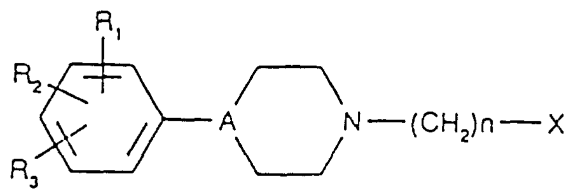

( VII )

dans laquelle
$R_1$ , $R_2$ , $R_3$, n et A ont la signification indiquée ci-dessus, avec un hétérocycle azoté de formule générale (III)

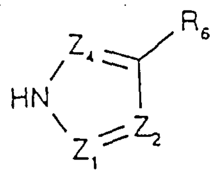

( III )

dans laquelle
$Z_1$, $Z_2$, $Z_4$ et $R_6$ ont la signification donnée ci-dessus.

La réaction est conduite dans une solution de diméthylsulfoxyde, diméthylformamide, un alcool comme l'éthanol, un hydrocarbure aromatique comme le toluène ou un hydrocarbure aliphatique comme l'hexane ou un éther comme le dioxanne. Cette réaction est conduite de préférence en présence d'une base comme le carbonate de potassium ou la triéthylamine.

La température de la réaction varie entre la température ambiante et la température de reflux du solvant utilisé.

Les temps de réaction varient entre 1 et 24 heures.

Procédé E :

Par déshydratation de composés de formule générale (I)

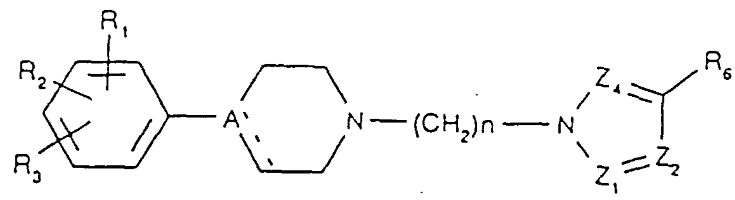

( I )

dans laquelle

$R_1$, $R_2$, $R_3$, n, $Z_1$, $Z_2$, $Z_4$ et $R_6$ ont la signification indiquée ci-dessus, A représente un atome de carbone uni à un groupe hydroxyle (C-OH) et la ligne pointillée représente un défaut de toute liaison additionnelle.

La réaction s'effectue en milieu acide comme par exemple l'acide chlorhydrique, l'acide trifluoroacétique, l'acide sulfurique ou phosphorique, ou par traitement avec du chlorure de thionyle dans le benzène.

La température de réaction varie entre la température ambiante et 180°C.

Les temps de réaction varient entre 1 et 14 heures.

Procédé F :

Par addition de réactifs organo-métalliques - par exemple phényl-lithium ou bromure de phénylmagnésium - à des composés de formule générale (VIII)

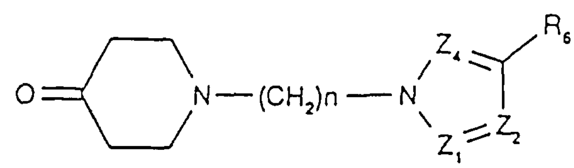

( VIII )

dans laquelle

n, $Z_1$, $Z_2$, $Z_4$ et $R_6$ ont la signification donnée ci-dessus.

La réaction est conduite dans une solution de solvant inerte, généralement un éther comme par exemple le diméthoxyéthane, le tétrahydrofuranne ou l'éther éthylique.

La température de réaction varie entre la température ambiante et la température de reflux du solvant utilisé.

Les temps de réaction varient entre 5 minutes et 24 heures.

Procédé G :

Par réduction du carbonyle du groupe amide de composés de formule générale (IX)

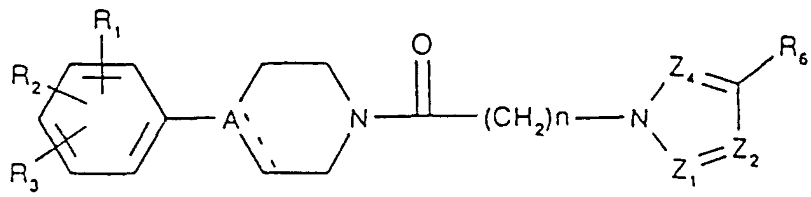

( IX )

dans laquelle
$R_1$, $R_2$, $R_3$, A, n, $Z_1$, $Z_2$, $Z_4$ et $R_6$ ont la signification indiquée ci-dessus.

La réaction est conduite de préférence dans un solvant organique inerte comme l'éther éthylique ou le tétrahydrofuranne avec des agents réducteurs comme $LiAlH_4$, $AlH_3$ ou le diborane.

La température de réaction varie entre la température ambiante et la température de reflux du solvant utilisé.

Les temps de réaction varient entre 5 minutes et 24 heures.

Procédé H :

Par réaction d'un composé de formule générale (I) avec un acide minéral ou organique non toxique dans un solvant adéquat, qui peut être par exemple un alcool comme le méthanol, l'éthanol ou l'un quelconque des propanols ou butanols, un ester comme l'acétate d'éthyle ou un nitrile comme l'acétonitrile ; et en utilisant les techniques habituelles de précipitation, cristallisation, etc., on obtient le sel correspondant.

On choisit l'acide minéral, entre autres, parmi les acides chlorhydrique, bromhydrique, sulfurique ou phosphorique ; et l'acide organique parmi les acides mono-, di- ou tricarboxyliques, comme par exemple les acides acétique, lactique, malonique, succinique, glutarique, fumarique, malique, tartrique, citrique, ascorbique, maléique, benzoïque, phénylacétique, cinnamique, salicylique et les acides alkyl-, cycloalkyl- ou arylsulfoniques.

On peut former les mono- ou disels de l'acide et ces sels peuvent être sous forme anhydre ou hydratée.

L'invention est précisée par les exemples suivants, donnés à simple titre de précision, étant entendu qu'en aucune manière ils ne peuvent limiter les conditions spécifiques du procédé ni la portée de l'invention.

**PROCEDE A**

Exemple 1. Préparation de 4-chloro-1-[4-(4-hydroxy-4-phényl-1-pipéridinyl)butyl]-1H-pyrazole.

On chauffe à reflux pendant 20 heures un mélange de 15,0 g (48 mmoles) de 8-hydroxy-8-phényl-5-azoniaspiro[4,5]décane, 5,4 g (53 mmoles) de 4-chloropyrazole et 13,2 g de carbonate de potassium dans 200 ml de diméthylformamide. Ensuite on fait évaporer à siccité à pression réduite, on redissout dans le chloroforme et on lave de façon répétée avec de l'eau. On sèche la phase organique avec du sulfate de sodium anhydre et on fait évaporer à pression réduite, obtenant un produit brut que l'on met en suspension dans l'éther éthylique, qu'on filtre à froid et qu'on lave à l'éther éthylique. On obtient 12,4 g (37,1 mmoles) de 4-chloro-1-[4-(4-hydroxy-4-phényl-1-pipéridinyl)butyl]-1H-pyrazole.

Le point de fusion et les données spectroscopiques pour l'identification de ce produit sont fournis au tableau 1.

**PROCEDE B**

Exemple 7. Préparation de 4-chloro-1-[4-[4-hydroxy-4-(3-trifluoro-méthylphényl)-1-pipéridinyl]butyl]-1H-pyrazole.

On chauffe à reflux pendant 4 heures un mélange de 8,6 g (35 mmoles) de 4-hydroxy-4-(3-trifluorométhylphényl)-1-pipéridine, 7,68 g de 1,4-dibromobutane et 13,8 g de carbonate de potassium dans 100 ml de diméthylformamide. Ensuite, on ajoute 3,59 g (35 mmoles) de 4-chloropyrazole et on maintient à reflux pendant 14 heures. Ensuite on fait évaporer à siccité à pression réduite, on redissout dans le chloroforme et on lave de façon répétée avec de l'eau. On sèche la phase organique avec du sulfate de sodium anhydre et on fait évaporer à pression réduite, obtenant un produit brut que l'on purifie par chromatographie sur gel de silice. On obtient 9,7 g (24,2 mmoles) de 4-chloro-1-{4-[4-hydroxy-4-(3-trifluorométhylphényl}-1-pipéridinyl]butyl}-1H-pyrazole.

Les données spectroscopiques pour l'identification de ce produit se trouvent au tableau I.

**PROCEDE C**

Exemple 14a. Préparation de 1-{4-[4-phényl-1-(1,2,3,6-tétrahydro-pyridinyl)]butyl}indole.

On chauffe à 90°C pendant 3 heures un mélange de 4,8 g (30 mmoles) de 4-phényl-1,2,3,6-tétrahydropyridine, 6,22 g de 1-(4-chlorobutyl)indole, 8,3 g de carbonate de potassium dans 100 ml de diméthylformamide. Ensuite on fait évaporer à siccité à pression réduite, on redissout dans le chloroforme et on lave de façon répétée avec de l'eau. On sèche la phase organique avec du sulfate de sodium anhydre et on fait évaporer à pression réduite, obtenant un produit brut qu'on purifie par chromatographie sur gel de silice. On obtient 5,7 g (17,3 mmoles) de 1-{4-[4-phényl-1-(1,2,3,6-tétrahydropyridinyl)]butyl}indole.

Les données spectroscopiques pour l'identification de ce produit se trouvent au tableau II.

**PROCEDE D**

Exemple 21a. Préparation de 4-chloro-1-{4-[4-phényl-1-(1,2,3,6-tétrahydropyridinyl)]propyl}-1H-pyrazole.

A une suspension de 1,0 g de NaH dans le diméthylformamide on ajoute, goutte à goutte, une solution de 2,05 g (20 mmoles) de 4-chloropyrazole dans le diméthylformamide. On chauffe la suspension blanche pendant 30 minutes à 100°C. On refroidit et on ajoute 4,7 g (20 mmoles) de 1-(3-chloropropyl)-4-phényl-1,2,3,6-tétrahydropyridine dissous dans le diméthylformamide. On chauffe à reflux pendant 2 heures. Ensuite on fait évaporer à siccité, on extrait le résidu avec du chloroforme, on lave avec de l'eau et on sèche avec du sulfate de sodium. On purifie le produit brut résultant par chromatographie sur gel de silice. On obtient 5,2 g (17,2 mmoles) de 4-chloro-1-{4-[4-phényl-1-(1,2,3,6-tétrahydro-pyridinyl)]propyl}-1H-pyrazole.

Les données spectroscopiques pour l'identification de ce produit sont fournies au tableau II.

**PROCEDE E**

Exemple 2a. Préparation de 1-{4-[4-phényl-1-(1,2,3,6-tétrahydro-pyridinyl)]butyl}-1H-benzimidazole.

On chaufffe à reflux pendant 6 heures une solution de 13,4 g (38,2 mmoles) de 1-[4-(4-hydroxy-4-phényl-1-pipéridinyl)butyl]-1H-benzimidazole, 150 ml d'HCl concentré et 75 ml d'éthanol. Ensuite on fait évaporer l'éthanol, on refroidit la solution aqueuse, on la rend basique avec NaOH dilué et on extrait avec du chloroforme. On sèche la phase organique avec du sulfate de sodium anhydre et on fait évaporer à pression réduite, obtenant un produit brut que l'on purifie par chromatographie sur gel de silice. On obtient 9,1 g (27,5 mmoles) de 1-{4-[4-phényl-1-(1,2,3,6-tétrahydropyridinyl)]butyl}-1H-benzimidazole.

Le point de fusion et les données spectroscopiques pour l'identification de ce produit sont fournis au tableau II.

**PROCEDE F**

Example 11. Préparation de 4,5-dichloro-1-{4-[4-hydroxy-4-(4-méthylphényl)-1-pipéridinyl]butyl}-2-méthyl-1H-imidazole.

A une suspension de 1,08 g (11,4 mmoles) de MgCl2 dans 15 ml de tétrahydrofuranne anhydre à - 40°C et sous une atmosphère d'azote, on ajoute une solution de 1,0 g (3,3 mmoles) de 4,5-dichloro-2-méthyl-1-{4-[4-oxo-1-pipéridiny]butyl}-1H-imidazole dans 10 ml de THF anhydre. On agite le mélange pendant 5 minutes puis, à - 40°C, on ajoute 6,8 ml d'une solution 1,0 M de bromure de 4-méthylphénylmagnésium. On agite la suspension résultante pendant 15 minutes à - 40°C et pendant 3 heures à la température ambiante. Ensuite on ajoute une solution aqueuse de chlorure d'ammonium et on fait évaporer le tétrahydrofuranne. On extrait la phase aqueuse résultante avec du chloroforme. On lave la phase chloroformique avec de l'eau, on sèche avec du sulfate de sodium anhydre, et on fait évaporer à siccité, obtenant un produit brut qu'on purifie par chromatographie sur gel de silice, donnant 1,02 g (2,6 mmoles) de 4,5-dichloro-1-{4-[4-hydroxy-4-(4-méthylphényl)-1-pipéridinyl]butyl}-2-méthyl-1H-imidazole.

Les données spectroscopiques pour l'identification de ce produit se trouvent au tableau I.

**PROCEDE G**

Exemple 16a. Préparation de 1-{4-[4-phényl-1-(1,2,3,6-tétrahydro-pyridinyl)]butyl}-1H-pyrazole.

A une solution de 3,3 g (10 moles) de 1-{4-[4-phényl-1-(1,2,3,6-tétrahydropyridinyl)]-3-oxo-butyl}-1H-pyrazole

dans 25 ml de THF on ajoute 2,0 g de LiAlH4. On fait refluer le mélange résultant durant 2 heures. On détruit l'excès de LiAlH4 par addition de NaOH concentré et d'eau. On filtre les sels inorganiques et on fait évaporer le THF sous vide, donnant 2,6 g (8,2 moles) de 1-{4-[4-phényl-1-(1,2,3,6-tétrahydropyridinyl)]butyl}-1H-pyrazole.

Le point de fusion et les données spectroscopiques pour l'identification de ce produit se trouvent au tableau II.

**PROCEDE H**

Exemple 11a. Préparation du chlorhydrate de 4,5-dichloro-2-méthyl-1-{4-[4-phényl-1-(1,2,3,6-tétrahydropyridinyl)]butyl}-1H-imidazole.

A une solution de 7,4 g (20,3 mmoles) de 4,5-dichloro-2-méthyl-1-{4-[4-phényl-1-(1,2,3,6-tétrahydropyridinyl)]butyl]-1H-imidazole dans 15 ml d'éthanol absolu refroidi dans un bain de glace, on ajoute 2,5 ml d'une solution d'éthanol/acide chlorhydrique 8,4 N. Au bout de quelques minutes apparait un précipité que l'on filtre, qu'on lave à l'éthanol froid et qu'on sèche, obtenant 7,7 g (19,2 mmoles) de chlorhydrate de 4,5-dichloro-2-méthyl-1-{4-[4-phényl-1-(1,2,3,6-tétrahydropyridinyl)]butyl}-1H-imidazole.

Le point de fusion et les données spectroscopiques pour l'identification de ce produit se trouvent au tableau II.

TABLEAU I

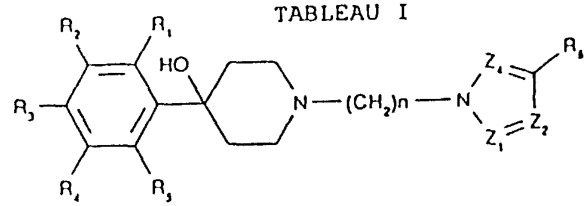

| Ex | $R_1$ | $R_2$ | $R_3$ | $R_4$ | $R_5$ | $Z_1$ | $Z_2$ | $R_6$ | $Z_4$ | n | P.F. | IR $cm^{-1}$ | $^1$H-RMN (300 MHz),δ (solvant) |
|---|---|---|---|---|---|---|---|---|---|---|---|---|---|
| 1 | H | H | H | H | H | N | CH | Cl | CH | 4 | 102-103°C | 3364 (b.a., OH), 2950, 2810, 1375, 1130, 991, 969, 760, 696, 605 KBr | 1,56 (quin,J=7,1Hz, 2H); 1,65 (b.a., 1H); 1,76 (d,J=12,4Hz, 2H); 1,90 (quin,J=7,6Hz, 2H); 2,20 (m, 2H); 2,40-2,55 (a.c., 4H); 2,83 (d,J=9,5Hz, 2H); 4,11 (t,J=7Hz, 2H); 7,21-7,42 (a.c., 5H); 7,52 (d,J=8,5Hz, 2H) ($CDCl_3$) |
| 2 | H | H | H | H | H | C-$CH_3$ | N | Cl | CCl | 4 | 86-89°CF | 3196 (b.a., OH), 2951, 2924, 2824, 1406, 1247, 1146, 762, 703 KBr | 1,59 (m,J=5,3 J'=6,6, 2H); 1,70-1,32 (a.c., 4H); 2,16 (d,t,J=13,0Hz J'=4,4Hz, 2H); 2,37 (s, 3H); 2,41-2,55 (a.c., 5H); 2,79 (d,J=11,3Hz, 2H); 3,88 (t,J=7,5Hz, 2H); 7,27 (t,J=7,2Hz, 1H); 7,36 (t,J=7,6Hz, 2H); 7,51 (d,J=7,3Hz, 2H) ($CDCl_3$) |
| 3 | H | H | H | H | H | CH | N | CH=CH-CH=CH-C | | 4 | 122-123°C | 3180 (b.a., OH), 2929, 2818, 1496, 1467, 1459, 1445, 1286, 1219, 1143, 769, 743, 707 KBr | 1,51 (quin,J=7,4Hz, 2H); 1,73 (d,J=12,7Hz, 2H); 1,87 (quin,J=7,6Hz, 2H); 2,10 (dt,J=12,9Hz J'=4,1Hz, 2H); 2,36-2,50 (a.c., 4H); 2,70 (d,J=11,2Hz, 2H); 3,25 (b.a., 1H); 4,12 (t,J=7,1Hz, 2H); 7,21-7,40 (a.c., 6H); 7,51 (d,J=8,3Hz, 2H); 7,70-7,75 (a.c., 2H) ($CDCl_3$) |
| 4 | H | H | H | H | H | CH | N | H | N | 4 | 123°C | 3180 (b.a., OH), 2949, 2919, 2838, 1276, 1145, 1135, 1006, 770, 707, 676 KBr | 1,45 (quin,J=7,5Hz, 2H); 1,69 (d,J=12,9Hz, 2H); 1,85 (quin,J=7,5Hz, 2H); 2,07 (dt,J=13,0Hz J'=4,1Hz, 2H); 2,33-2,45 (a.c., 4H); 2,69 (d,J=11,2Hz, 2H); 2,93 (b.a., 1H); 4,10 (t,J=6,9Hz, 2H); 7,18 (t,J=7Hz, 1H); 7,27 (t,J=7,8Hz, 2H); 7,46 (d,J=8,3Hz, 2H); 7,80 (s, 1H); 7,91 (s, 1H) ($CDCl_3$) |

TABLEAU I (suite)

| Ex. | $R_1$ | $R_2$ | $R_3$ | $R_4$ | $R_5$ | $Z_1$ | $Z_2$ | $R_6$ | $Z_4$ | n | P.F. | IR $cm^{-1}$ | $^1H$-RMN (300 MHz),δ (solvant) |
|---|---|---|---|---|---|---|---|---|---|---|---|---|---|
| 5 | H | H | Cl | H | H | N | CH | Cl | CH | 4 | 106°C | 3145 (b.a., OH), 2947, 2918, 2834, 1318, 1147, 1083, 1112, 990, 817, 612 KBr | 1,47 (quin,J=7,5Hz, 2H); 1,69 (d,J=11,9Hz, 2H); 1,84 (quin, J= 7,6Hz, 2H); 2,05 (dt,J=13Hz, J'=4,4Hz, 2H); 2,34-2,50 (a.c., 5H); 2,72 (d,J=11,2Hz, 2H); 4,05 (t,J=7.0Hz, 2H); 7,29 (syst. AB,J=8,6Hz, 2H); 7,36 (s, 2H); 7,42 (syst AB,J=8,6Hz, 2H) ($CDCl_3$) |
| 6 | H | H | Cl | H | H | C-$CH_3$ | N | Cl | CCl | 4 | huile | 3340 (b.a., OH), 2946, 2820, 1537, 1492, 1471, 1406, 1376, 1247, 1135, 1094, 1013, 828, 755 film | 1,54 (m, 2H); 1,67-1,78 (a.c., 4H); 2,06 (dt,J=13Hz, J'=4,2Hz, 2H); 2,32 (s, 3H); 2,38-2,45 (a.c., 5H); 2,73 (d,J=11,2Hz, 2H); 3,86 (t,J=7,3Hz, 2H); 7,28 (syst AB, J=8,6Hz, 2H); 7,43 (syst AB,J=8,6Hz, 2H) ($CDCl_3$) |
| 7 | H | $CF_3$ | H | H | H | N | CH | Cl | CH | 4 | huile | 3360 (b.a., OH), 2948, 2823, 1438, 1378, 1330, 1212, 1165, 1124, 1047, 972, 804, 704 film | 1,48 (quin,J=7,6Hz, 2H); 1,71 (d,J=12,5Hz, 2H); 1,85 (quin,J=7,6Hz, 2H); 2,06-2,21 (a.c., 3H); 2,36-2,43 (a.c., 4H); 2,76 (d,J=11,5Hz, 2H); 4,06 (t,J=7,1Hz, 2H); 7,35 (s, 2H); 7,43-7,51 (a.c., 2H); 7,66 (d,J=7,5Hz, 1H); 7,79 (s, 1H) ($CDCl_3$) |
| 8 | H | $CF_3$ | H | H | H | C-$CH_3$ | N | Cl | CCl | 4 | huile | 3340 (b.a., OH), 2948, 2823, 1408, 1330, 1165, 1126, 1075, 789, 763, 704 film | 1,57 (quin,J=7,5Hz, 2H); 1,70-1,80 (a.c., 4H); 2,15 (dt, J=12,9Hz J'=3,6Hz, 2H); 2,35 (s, 3H); 2,40-2,52 (a.c., 4H); 2,80 (d,J=11,7Hz, 2H); 3,88 (t,J=7,0Hz, 2H); 7,42-7,57 (a.c., 2H); 7,69 (d,J=7,5Hz, 1H); 7,82 (s, 1H) ($CDCl_3$) |

TABLEAU I (suite)

| Ex | $R_1$ | $R_2$ | $R_3$ | $R_4$ | $R_5$ | $Z_1$ | $Z_2$ | $R_6$ | $Z_4$ | n | P.F. | IR $cm^{-1}$ | $^1H$-RMN (300 MHz),δ (solvant) |
|---|---|---|---|---|---|---|---|---|---|---|---|---|---|
| 9 | H | H | F | H | H | C-$CH_3$ | N | Cl | CCl | 4 | huile | 3330 (b.a., OH), 2946, 2818, 1509, 1406, 1247, 1222, 1160, 835 film | 1,58 (m, 2H); 1,64-1,81 (a.c., 4H); 2,14 (dt,J=12,9Hz J'=3,6Hz 2H); 2,32 (s, 3H); 2,43-2,60 (a.c., 4H); 2,84 (d,J=11Hz, 2H); 3,87 (t,J=7,1Hz, 2H); 4,18 (b.a., 1H); 7,01 (t,J=8,8Hz, 2H); 7,46 (dd,J=8,8Hz J'=5,2Hz, 2H) ($CDCl_3$) |
| 10 | H | H | H | H | H | CH | CH | CH=CH-CH=CH-C | | 4 | 109-111°C | 3190 (b.a., OH), 2956, 2823, 1461, 1446, 1319, 1303, 1218, 1142, 738, 703 KBr | 1,57 (m, 2H); 1,73 (d,J=14Hz, 2H); 1,80 (b.a., 1H); 1,90 (m, 2H); 2,13 (dt,J=13Hz J'=4Hz, 2H); 2,32-2,46 (a.c., 4H); 2,76 (d,J=11,3Hz, 2H); 4,16 (t,J=7,1Hz, 2H); 6,50 (d,J=3,1Hz, 1H); 7,05-7,14 (a.c., 2H); 7,18-7,40 (a.c., 5H); 7,50 (d,J=7,8Hz, 2H); 7,00 (d,J=7,3Hz, 1H) ($CDCl_3$) |
| 11 | H | H | $CH_3$ | H | H | C-$CH_3$ | N | Cl | CCl | 4 | huile | 3360 (b.A., OH) 2946, 2818, 1535, 1471, 1406, 1376, 1247, 1134, 817, 755 film | 1,53 (m, 2H); 1,66-1,84 (a.c., 4H); 2,09 (dt,J=12,9Hz, J'=3,6Hz, 2H); 2,33 (s, 3H); 2,36 (s,3H); 2,39-2,50 (a.c., 4H); 2,77 (d,J=11,2Hz, 2H); 3,87 (t,J=7,0Hz, 2H); 7,15 (syst AB,J=7,8Hz, 2H); 7,33 (syst AB,J=7,8Hz, 2H) ($CDCl_3$) |
| 12 | H | H | H | H | H | N | CH | H | CH | 4 | 89-91°C | 3137 (b.A., OH) 2947, 2532, 1396, 1378, 1119, 1046, 756, 697 KBr | 1,51 (quin,J=7,6Hz,2H); 1,73 (d,J=12,3Hz, 2H); 1,89 (quin,J=7,6Hz, 2H); 2,00-2,20 (a.c., 3H); 2,35-2,45 (a.c., 4H); 2,76 (d,J=10,2Hz, 2H); 4,13 (t,J=7,1Hz, 2H); 6,21 (s, 1H); 7,21 (m, 1H); 7,30-7,37 (a.c., 3H); 7,44-7,52 (a.c., 3H) ($CDCl_3$) |

TABLEAU I (suite)

| Ex | $R_1$ | $R_2$ | $R_3$ | $R_4$ | $R_5$ | $Z_1$ | $Z_2$ | $R_6$ | $Z_4$ | n | P.F. | IR $cm^{-1}$ | $^1H$-RMN (300 MHz),δ (solvant) |
|---|---|---|---|---|---|---|---|---|---|---|---|---|---|
| 13 | H | H | H | H | H | N | CH | CH=CH-CH=CH-C | | 4 | 107-109°C | 3311 (b.a., OH) 2953, 2803, 1465, 1375, 1133, 1117, 1043, 1017, 761, 744, 704 KBr | 1,53 (m, 2H); 1,71 (d,J=12,2Hz, 2H); 1,95 (m, 2H); 2,10 (m, 2H); 2,29 (b.a., 1H); 2,35-2,47 (a.c., 4H); 2,71 (d,2H); 4,39 (t,J=7,1Hz, 2H); 7,13 (t, 1H); 7,22-7,44 (a.c., 5H); 7,50 (d,J=8Hz, 2H); 7,71 (d,J=8,3Hz, 1H); 7,95 (s, 1H) ($CDCl_3$) |
| 14 | H | - H | H | H | H | N | C-CH=CH-CH=CH | | CH | 4 | 120-122°C | 3295 (b.a., OH) 2946, 2817, 1377, 1126, 786, 735, 700 KBr | 1,58 (m, 2H); 1,73 (d,J=13,5Hz, 2H); 1,90-2,20 (a.c., 5H); 2,38-2,47 (a.c., 4H); 2,75 (d,J=10,5Hz, 2H); 4,42 (t,J=6,6Hz, 2H); 7,06 (t,J=7,5Hz, 1H); 7,22-7,37 (a.c., 4H); 7,49 (d, J=7,8Hz, 2H); 7,61-7,71 (a.c., 2H); 7,90 (s, 1H) ($CDCl_3$) |
| 15 | H | H | $CH_3$ | H | H | N | CH | Cl | CH | 4 | 81-82°C | 3122 (b.a., OH) 2936, 1475, 1434, 1378, 1319, 989, 973, 814 KBr | 1,51 (quin,J=7,6Hz, 2H); 1,73 (d,J=11,7Hz, 2H); 1,87 (quin, J=7,6Hz, 2H); 2,12 (dt,J=12,8Hz J'=4,4Hz, 2H); 2,33 (s, 3H); 2,35-2,48 (a.c., 5H); 2,74 (d,J=11,2Hz, 2H); 4,07 (t,J=7,1Hz, 2H); 7,15 (d,J=8Hz, 2H); 7,25-7,40 (a.c., 4H) ($CDCl_3$) |
| 16 | H | H | $CH_3O$ | H | H | N | CH | Cl | CH | 4 | 122-123°C | 3190 (b.a., OH) 2954, 2923, 2827, 1509, 1314, 1243, 1178, 971 | 1,49 (quin,J=7,6Hz, 2H); 1,72 (d,J=11,8Hz, 2H); 1,84 (quin,J=7,4Hz, 2H); 2,00-2,14 (a.c. (dt+b.a.), 3H); 2,34-2,47 (a.c., 4H); 2,72 (d,J=11Hz, 2H); 3,77 (s, 3H); 4,05 (t,J=7,1Hz, 2H); 6,85 (d,J=9Hz, 2H); 7,24-7,42 (a.c., 4H) ($CDCl_3$) |
| 17 | H | H | H | :H | H | CPh | N | H | CH | 4 | 108-110°C | 3220 (b.a., OH) 2944, 2817, 1473, 1446, 1421, 1136, 1046, 787, 773, 761, 700 film | 1,45 (quin,J=7,6Hz, 2H); 1,68-1,82 (a.c., 4H); 2,08 (dt,J=13,0Hz J'=4,1Hz, 2H); 2,29-2,42 (a.c., 4H); 2,51 (b.a.,1H); 2,67 (d,J=11,2Hz, 2H); 4,01 m(t,J=7,3Hz, 2H); 7,01(s,1H); 7,08 (s, 1H); 7,20-7,56 (a.c., 10H) ($CDCl_3$) |

TABLEAU I (suite)

| Ex | $R_1$ | $R_2$ | $R_3$ | $R_4$ | $R_5$ | $Z_1$ | $Z_2$ | $R_6$ | $Z_4$ | n | P.F. | IR $cm^{-1}$ | $^1H$-RMN (300 MHz),δ (solvant) |
|---|---|---|---|---|---|---|---|---|---|---|---|---|---|
| 18 | H | H | $CH_3$ | H | H | CH | N | CH=CH-CH=CH-C | | 4 | huile | 3260 (b.a., OH) 2944, 2817, 1497, 1459, 1381, 1287, 1135, 1046, 817, 745 film | 1,58 (quin,J=7,6Hz, 2H); 1,74 (d,J=12Hz, 2H); 1,82 (b.a., 1H); 1,95 (quin,J=7,6Hz, 2H); 2,11 (dt, 2H); 2,33 (s,3H); 2,40-2,50 (a.c., 4H); 2,74 (d,J=11,5Hz, 2H); 4,20 (t,J=7,1Hz, 2H); 7,15 (d,J=8,3Hz, 2H); 7,22-7,35 (a.c., 3H); 7,37-7,43 (a.c., 2H); 7,79 (m, 1H); 7,87 (s, 1H) ($CDCl_3$) |
| 19 | H | H | H | H | H | CH | N | Ph | CPh | 4 | 138-139°C | 3194 (b.a., OH) 2939, 2806, 1509, 1446, 773, 766, 758, 696 KBr | 1,38 (m, 2H); 1,56 (m, 2H); 1,72 (d,J=12,4Hz, 2H); 2,09 (dt, 2H); 2,25 (t,J=7,4Hz, 2H); 2,39 (m, 2H); 2,66 (m,2H); 3,10 (b.a., 1H); 3,78 (t,J=7,2Hz, 2H); 7,10-7,52 (a.c., 16H); |
| 20 | CH=CH-CH=CH | | H | H | H | N | CH | Cl | CH | 4 | huile | 3357 (b.a., OH), 2946, 2833, 1434, 1379, 1315, 1140, 1123, 972, 781, 613 KBr | 1,44 (quin,J=7,3Hz, 2H); 1,77 (quin,J=7,5Hz, 2H); 2,15-2,30 (a.c., 5H); 2,34 (t,J=7,5Hz, 2H); 2,57 (m, 2H); 2,73 (d,J=11,3Hz, 2H); 3,99 (t,J=7,1Hz, 2H); 7,26-7,46 (a.c., 6H); 7,73 (d,J=8,1Hz, 1H); 7,82 (m,1H); 8,91 (m, 1H) ($CDCl_3$) |
| 21 | H | CH=CH-CH=CH | | H | H | N | CH | Cl | CH | 4 | 142-143°C | 3131 (b.a., OH), 2950, 2820, 1377, 1311, 971, 829, 761, 613 KBr | 1,55 (quin,J=7,5Hz, 2H); 1,70-1,97 (a.c., 5H); 2,29 (dt,J=12,7Hz, J'=4,1Hz, 2H); 2,41-2,55 (a.c., 4H); 2,83 (d,J=11,7Hz, 2H); 4,11 (t,J=7,0Hz, 2H); 7,39-7,50 (a.c., 4H); 7,64 (dd,J=9,1Hz,J'=1,5Hz, 1H); 7,81-7,85 (a.c., 3H); 7,95 (s, 1H) ($CDCl_3$) |

TABLEAU I (suite)

| Ex | $R_1$ | $R_2$ | $R_3$ | $R_4$ | $R_5$ | $Z_1$ | $Z_2$ | $R_6$ | $Z_4$ | n | Sel / P.F. | IR $cm^{-1}$ | $^1$H-RMN (300 MHz),δ,J=Hz (solvant) |
|---|---|---|---|---|---|---|---|---|---|---|---|---|---|
| 22 | H | H | H | H | H | N | CH | 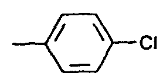 | CH | 4 | 137-140°C | 3347, 2944, 2810, 1562, 1492, 1376, 1127, 1094, 1002, 952, 828, 760, 699 KBr | 1,56 (m, 2H); 1,74 (m, 2H); 1,80 (b.a, 1H); 1,94 (m, 2H); 2,40 (dt,J=13,1Hz,J'=4,0Hz, 2H); 2,40-2,50 (a.c., 4H); 2,77 (m, 2H); 4,15 (t,J=7,0Hz, 2H); 7,25-7,40 (a.c., 7H); 7,50 (d,J=8,3Hz, 2H); 7,61 (s, 1H); 7,72 (s,1H) ($CDCl_3$) |
| 23 | H | H | F | H | H | CH | N | CH=CH-CH=CH-C | | 4 | 120-122°C | 3230, 2947, 2915, 1504, 1219, 1135, 835, 746 KBr | 1,58 (m, 2H); 1,70 (m, 2H); 1,93 (m, 2H); 2,12 (m, 2H); 2,40-2,55 (a.c., 4H); 2,76 (m, 2H); 4,19 (t,J=7,0Hz, 2H); 7,02 (m, 2H); 7,26 (m, 2H); 7,30-7,50 (a.c., 3H); 7,74 (m, 1H); 7,83 (s, 1H) ($CDCl_3$) |
| 24 | H | $CCF_3$ | H | H | H | N | CH | Cl | CH | 4 | HCl 147-148°C | 3259, 2465, 2420, 2365, 1328, 1108, 1073 KBr | 1,62-1,84 (a.c., 6H); 2,53 (m, 2H); 3,09-3,40 (a.c., 6H); 4,12 (t,J=6,8Hz, 2H); 5,76 (s, 1H); 7,51 (s, 1H); 7,52-7,82 (a.c., 4H); 8,02 (s, 1H); 10,96 (b.a., 1H) (DMSO-$d_6$) |
| 25 | H | H | F | H | H | N | CH | CH=CH-CH=CH-C | | 4 | 136-137°C | 3303, 2951, 2805, 1506, 1464, 1376, 1218, 1162, 1118, 832, 741 KBr | 1,54 (m, 2H); 1,60-1,80 (a.c., 3H); 1,97 (m, 2H); 2,06 (dt,J=13,0Hz, J'=4,3Hz, 2H); 2,30-2,43 (a.c. 4H; 2,72 (m, 2H); 4,40 (t,J=7,0Hz, 2H); 6,99 (t,J=8,8Hz, 2H); 7,12 (m, 1H); 7,32-7,47 (a.c., 4H); 7,71 (d,J=8,1Hz, 1H); 7,96 (s, 1H) ($CDCl_3$-$CD_3OD$ [1:1]) |
| 26 | H | H | F | H | H | N | C-CH=CH-CH=CH | | CH | 4 | 148-150°C | 3325, 2950, 2923, 2812, 1509, 1377, 1218, 1131, 834, 758 KBr | 1,57 (m, 2H); 1,70-1,77 (a.c., 3H); 1,98-2,19 (a.c., 4H); 2,35-2,49 (a.c., 4H); 2,77 (d,J=11,2Hz, 2H); 4,45 (t,J=7,0Hz, 2H); 6,98-7,15 (a.c., 3H); 7,25-7,49 (a.c., 3H); 7,63 (d,J=8,3Hz, 1H); 7,69 (d,J=7,8Hz, 1H); 7,91 (s, 1H) ($CDCl_3$-$CD_3OD$ [1:1]) |

TABLEAU I (suite)

| Ex | $R_1$ | $R_2$ | $R_3$ | $R_4$ | $R_5$ | $Z_1$ | $Z_2$ | $R_6$ | $Z_4$ | n | Sel / P.F. | IR $cm^{-1}$ | $^1H$-RMN (300 MHz),δ,J=Hz (solvant) |
|---|---|---|---|---|---|---|---|---|---|---|---|---|---|
| 27 | H | H | F | H | H | N | C-CH=CH-CH=CH | | N | 4 | 109-110°C | 3400, 2931, 2812, 1509, 1229, 1101, 831, 745 KBr | 1,47-1,80 (a.c., 4H); 1,90-2,25 (a.c., 5H); 2,30-2,55 (a.c., 4H); 2,70 (m, 2H); 4,78 (t,J=6,9Hz, 2H); 7,01 (t,J=8,7Hz, 2H); 7,26-7,54 (a.c., 4H); 7,85 (dd,J=6,7Hz, J'=3,0Hz, 2H) ($CDCl_3$-$CD_3OD$ [1:1]) |
| 28 | H | H | F | H | H | N | N | CH=CH-CH=CH-C | | 4 | 102-103°C | 3430, 2952, 2925, 1508, 1223, 1140, 833, 744 KBr | 1,45-1,80 (a.c., 4H); 1,85-2,25 (a.c., 5H); 2,25-2,55 (a.c., 4H); 2,77 (m, 2H); 4,69 (t,J=6,9Hz, 2H); 7,01 (t,J=8,7Hz, 2H); 7,26-7,53 (a.c., 5H); 8,06 (d,J=7,3Hz, 1H) ($CDCl_3$-$CD_3OD$ [1:1]) |

TABLEAU II

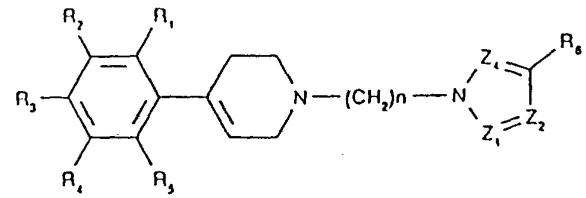

| Ex | $R_1$ | $R_2$ | $R_3$ | $R_4$ | $R_5$ | $Z_1$ | $Z_2$ | $R_6$ | $Z_4$ | n | P.F. | IR $cm^{-1}$ | $^1$H-RMN (300 MHz),δ (solvant) |
|---|---|---|---|---|---|---|---|---|---|---|---|---|---|
| 1a | H | H | H | H | H | N | CH | Cl | CH | 4 | 62-64°C | 3113, 2920, 2745, 1375, 1325, 1138, 965, 837, 742, 688 KBr | 1,56 (quin,J=7,6Hz,2H); 1,91 (quin,J=7,6Hz, 2H); 2,47 (t,J=7,4Hz, 2H); 2,58 (m, 2H); 2,65 (t,J=5,6Hz, 2H); 3,14 (m,2H); 4,11 (t,J=7,1Hz, 2H); 6,06 (m,1H); 7,23-7,42 (a.c., 7H) ($CDCl_3$) |
| 2a | H | H | H | H | H | CH | N | CH=CH-CH=CH-C | | 4 | 66-69°C | 2933, 1495, 745, 694, 665 film | 1,55 (quin,J=7,6Hz, 2H); 1,92 (quin,J=7,6Hz, 2H); 2,43 (t,J=7,3Hz, 2H); 2,52 (m, 2H); 2,61 (t,J=5,6Hz, 2H); 3,07 (m, 2H); 4,14 (t,J=7,1Hz, 2H); 6,02 (m, 1H); 7,20-7,40 (a.c., 8H); 7,80 (m, 1H); 7,86 (s, 1H) ($CDCl_3$) |
| 3a | H | H | H | H | H | CH | N | H | N | 4 | 63-64°C | 2942, 1438, 1381, 1271, 1142, 1006, 753, 697, 681, KBr | 1,56 (m, 2H); 1,95 (m, 2H); 2,47 (t,J=7,1Hz, 2H); 2,56 (m, 2H); 2,66 (t,J=5,3Hz, 2H); 3,11 (m, 2H); 4,19 (t,J=7,0Hz, 2H); 6,05 (s, 1H); 7,21 (m, 1H); 7,30 (t,J=7,6Hz, 2H); 7,36 (d,J=7,8Hz, 2H); 7,94 (s, 1H); 8,06 (s,1H) ($CDCl_3$) |
| 4a | H | H | Cl | H | H | N | CH | Cl | CH | 4 | 103-104°C | 2939, 1493, 1436, 1381, 1306, 1122, 1097, 973, 843, 824, 730 KBr | 1,54 (m, 2H); 1,90 (m, 2H); 2,45 (t,J=7,4Hz, 2H); 2,51 (m, 2H); 2,65 (t,J=5,6Hz, 2H); 3,10 (m, 2H); 4,10 (t,J=7,0Hz, 2H); 6,03 (m, 1H); 7,26 (syst AB,J=8,6Hz, 2H); 7,29 ( syst AB,J=8,6Hz, 2H); 7,37 (s, 1H); 7,41 (s, 1H) ($CDCl_3$) |
| 5a | H | H | Cl | H | H | C-$CH_3$ | N | Cl | CCl | 4 | 119-120°C | 2922, 1531, 1494, 1469, 1403, 1380, 1366, 1245, 1094, 1010 KBr | 1,59 (m,2H); 1,76 (m, 2H); 2,36 (s, 3H); 2,42-2,53 (a.c., 4H); 2,67 (t,J=5,3Hz, 2H); 3,12 (m, 2H); 3,88 (t,J=7,4Hz, 2H); 6,04 (m, 1H); 7,27 ( syst AB,J=9,1Hz, 2H); 7,30 (syst AB,J=9,1Hz, 2H) ($CDCl_3$) |

TABLEAU II (suite)

| Ex | $R_1$ | $R_2$ | $R_3$ | $R_4$ | $R_5$ | $Z_1$ | $Z_2$ | $R_6$ | $Z_4$ | n | P.F. | IR $cm^{-1}$ | $^1$H-RMN (300 MHz),δ (solvant) |
|---|---|---|---|---|---|---|---|---|---|---|---|---|---|
| 6a | H | $CF_3$ | H | H | H | N | CH | Cl | CH | 4 | huile | 2944, 1434, 1375, 1331, 1247, 1165, 1126, 1076, 972, 800, 698 film | 1,53, (quin,J=7,5Hz, 2H); 1,89 (quin,J=7,7Hz, 2H); 2,45 (t,J=7,3Hz, 2H); 2,54 (m, 2H); 2,66 (t,J=5,5Hz, 2H); 3,10 (m, 2H); 4,08 (t,J=7,1Hz, 2H); 6,10 (m, 1H); 7,35-7,56 (a.c., 5H); 7,59 (s, 1H) ($CDCl_3$) |
| 7a | H | $CF_3$ | H | H | H | C-$CH_3$ | N | Cl | CCl | 4 | huile | 2931, 2815, 1533, 1405, 1331, 1246, 1165, 1125, 1076, 797, 699 film | 1,62 (quin,J=6,6Hz, 2H); 1,77 (quin,J=7,6Hz, 2H); 2,37 (s, 3H); 2,51 (t,J=7,2Hz, 2H); 2,60 (m, 2H); 2,71 (t,J=5,6Hz, 2H); 3,17 (m, 2H); 3,89 (t,J=7,3Hz, 2H); 6,14 (m, 1H); 7,40-7,50 (a.c., 2H); 7,55 (d,J=7,5Hz, 1H); 7,62 (s, 1H) ($CDCl_3$) |
| 8a | H | H | F | H | H | N | CH | Cl | CH | 4 | 86-87°C | 2936, 1512, 1378, 1326, 1229, 988, 967 KBr | 1,60 (quin,J=7,5Hz, 2H); 1,91 (quin,J=7,5Hz, 2H); 2,50-2,82 (a.c., 4H); 2,76 (t,J=5,6Hz, 2H); 3,19 (m, 2H); 4,11 (t,J=6,9Hz, 2H); 5,97 (s, 1H); 6,99 (t,J=8,8Hz, 2H); 7,32 (dd,J=8,8Hz J'=5,4Hz, 2H); 7,38 (s, 1H); 7,40 (s,1H) ($CDCl_3$) |
| 9a | H | H | F | H | H | C-$CH_3$ | N | Cl | CCl | 4 | 79-82°C | 2934, 1531, 1512, 1408, 1247, 1225, 1167, 818 KBr | 1,59(m,2H); 1,76(m,2H); 2,37(s,3H);2,48(t,J=7,2Hz,2H); 2,54(m, 2H);2,67(t,J=5,6Hz,2H); 3,12(m, 2H);3,89 (t,J=7,3Hz,2H); 5,99(m, 1H);6,99(t,J=8,7Hz,2H); 7,33 (dd,J= 8,7Hz);J'=5,4Hz,2H) ($CDCl_3$) |
| 10a | H | H | H | H | H | C-$CH_3$ | N | Cl | CCl | 4 | huile | 2929, 1533, 1405, 1246, 748 film | 1,59 (m, 2H); 1,76 (m, 2H); 2,37 (s, 3H); 2,49 (t,J=7,3Hz, 2H); 2,58 (m, 2H); 2,69 (t,J=5,4Hz, 2H); 3,14 (m, 2H); 3,89 (t,J=7,4Hz, 2H); 6,06 (m, 1H); 7,22-7,40 (a.c., 5H) ($CDCl_3$) |
| 11a | H | H | H | H | H | C-$CH_3$ | N | Cl | CCl | 4 | · HCl 203-204°C | 2930, 2576, 1407, 1376, 1245, 750, KBr | 1,69 (m, 2H); 1,81 (m, 2H); 2,35 (s, 3H); 2,71 (d,J=7,2Hz, 1H); 2,91 (m, 1H); 3,17 (a.c., 3H); 3,56 (m, 1H); 3,75 (m, 1 H); 3,90-3,97 (a.c., 3H); 6,17 (s, 1H); 7,25-7,40 (a.c., 3H); 7,47 (d,J=7,6Hz, 2H); 11,30 (b.a., 1H) (DMSO-$d_6$). |

TABLEAU II (suite)

| Ex | $R_1$ | $R_2$ | $R_3$ | $R_4$ | $R_5$ | $Z_1$ | $Z_2$ | $R_6$ | $Z_4$ | n | P.F. | IR $cm^{-1}$ | $^1H$-RMN (300 MHz),δ (solvant) |
|---|---|---|---|---|---|---|---|---|---|---|---|---|---|
| 12a | H | H | H | H | H | C-$CH_3$ | N | Cl | CCl | 4 | • 2HCl 192-194°C | 3569, 2941, 2692, 2556, 1601, 1446, 769, 753, 698 KBr | 1,67 (m, 2H); 1,79 (m, 2H); 2,36 (s, 3H); 2,69 (d,J= 18,0Hz, 1H); 2,88 (m, 1H); 3,15 (a.c., 3H); 3,54 (m, 1H); 3,72 (m, 1H); 3,85-3,98 (a.c., 3H); 6,15 (s, 1H); 7,22-7,38 (a.c., 3H); 7,45 (d,J=7,3Hz, 2H); 9,93 (b.a., 1H); 11,36 (b.a., 1H) (DMSO-$d_6$) |
| 13a | H | -H | F | H | H | CH | CH | CH=CH-CH=CH-C | | 4 | huile | 2937, 1510, 1464, 1230, 1161, 816, 742 film | 1,61 (quin,J=7,7Hz, 2H); 1,93 (quin,J=7,6Hz, 2H); 2,42-2,58 (a.c., 4H); 2,66 (t,J=5,6Hz, 2H); 3,11 (m, 2H); 4,17 (t,J=7,0Hz, 2H); 5,98 (m, 1H); 6.51 (d,J=3,9Hz, 1H); 6,95-7,39 (a.c., 8H); 7,65 (d,J=7,8Hz, 1H) ($CDCl_3$) |
| 14a | H | H | H | H | H | CH | CH | CH=CH-CH=CH-C | | 4 | huile | 2938, 1510, 1485, 1463, 1446, 1376, 1336, 1315, 763, 740, 695 film | 1,63 (quin,J=7,4Hz, 2H); 1,94 (quin,J=7,4Hz, 2H); 2,49 (t,J=7,6Hz, 2H); 2,60 (m, 2H); 2,69 (t,J=5,3Hz, 2H); 3,14 (m, 2H); 4,19 (t,J=7,1Hz, 2H); 6,08 (m, 1H); 6,53 (m, 1H); 7,08-7,44 (a.c., 9H); 7,67 (d,J=8,1Hz, 1H) ($CDCl_3$) |
| 15a | H | H | $CH_3$ | H | H | C-$CH_3$ | N | Cl | CCl | 4 | 87-88°C | 2939, 2916, 1529, 1404, 1378, 1243, 1166, 1131, 1016 film | 1,59 (m, 2H); 1,75 (m, 2H); 2,32 (s, 3H); 2,36 (s, 3H); 2,47 (t,J=7,2Hz, 2H); 2,54 (m, 2H); 2,67 (t,J=5,2Hz, 2H); 3,11 (m, 2H); 3,87 (t,J=7,3Hz, 2H); 6,01 (s, 1H); 7,11 (syst AB,J=8,1Hz, 2H); 7,27 (syst AB,J=8,1Hz, 2H) ($CDCl_3$) |
| 16a | H | H | H | H | H | N | CH | H | CH | 4 | 36-38°C | 2941, 1396, 748, 695 film | 1,54 (quin,J=7,6Hz, 2H); 1,91 (quin,J=7,6Hz, 2H); 2,45 (t,J=7,6Hz, 2H); 2,55 (m, 2H); 2,65 (t,J=5,6Hz, 2H); 3,11 (m,2H); 4,14 (t,J=7,1Hz, 2H); 6,03 (m, 1H); 6,21 (m, 1H); 7,20-7,39 (a.c., 6H); 7,49 (m, 1H) ($CDCl_3$) |
| 17a | H | H | H | H | H | N | CH | CH=CH-CH=CH-C | | 4 | 50-52°C | 2942, 1465, 1158, 832, 740, 691 KBr | 1,61 (quin, 2H); 2,00 (quin,J=7,5Hz, 2H); 2,43-2,58 (a.c., 4H); 2,68 (m, 2H); 3,14 (s, 2H); 4,43 (t,J=6,6Hz, 2H); 6,02 (s, 1H); 7,13 (t,J=7,3Hz, 1H); 7,20-7,51(a.c., 7H); 7,73 (d,J=7,9Hz, 1H); 7,99 (s, 1H) ($CDCl_3$) |

TABLEAU II (suite)

| Ex | $R_1$ | $R_2$ | $R_3$ | $R_4$ | $R_5$ | $Z_1$ | $Z_2$ | $R_6$ | $Z_4$ | n | P.F. | IR $cm^{-1}$ | $^1H$-RMN (300 MHz),δ (solvant) |
|---|---|---|---|---|---|---|---|---|---|---|---|---|---|
| 18a | H | H | H | H | H | N | C-CH=CH-CH=CH | | CH | 4 | 73-75°C | 3049, 2940, 2778, 1467, 1371, 1158, 1143, 1131, 757, 742, 692 KBr | 1,60 (quin,J=7,6Hz, 2H); 2,09 (quin,J=7,4Hz, 2H); 2,48 (t,J=7,4Hz, 2H); 2,55 (m, 2H); 2,66 (t,J=5,6Hz, 2H); 3,11 (d,J=2,9Hz, 2H); 4,45 (t,J=7,1Hz, 2H); 6,03 (s, 1H); 7,07 (t,J=7,5Hz, 1H); 7,20-7,39 (a.c., 6H); 7,63 (d,J=4,3Hz, 1H); 7,70 (d,J=8Hz, 1H); 7,91 (s, 1H) ($CDCl_3$) |
| 19a | H | H | $CH_3$ | H | H | N | CH | Cl | CH | 4 | 72-73°C | 3115, 2938, 2740, 1376, 1328, 1137, 986, 966, 844, 824, 797 KBr | 1,55 (quin, 2H); 1,90 (quin,J=7,5Hz, 2H); 2,33 (s, 3H); 2,46 (t,J=7,5Hz, 2H); 2,55 (m, 2H); 2,66 (t,J=6,4Hz, 2H); 3,11 (m, 2H); 4,10 (t,J=7,0Hz, 2H); 6,01 (s, 1H); 7,12 (syst AB,J=8Hz, 2H); 7,27 (syst AB,J=8Hz, 2H); 7,37 (s, 1H); 7,41 (s, 1H) ($CDCl_3$) |
| 20a | H | H | $CH_3O$ | H | H | N | CH | Cl | CH | 4 | 104-105°C | 2923, 1533, 1405, 1379, 1246, 749 KBr | 1,54 (quin, 2H); 1,89 (quin,J=7,6Hz, 2H); 2,44 (t,J=7,4Hz, 2H); 2,52 (m, 2H); 2,65 (t,J=5,3Hz, 2H); 3,10 (m, 2H); 3,78 (s, 3H); 4,09 (t,J=7,0Hz, 2H); 5,95 (s, 1H); 6,84 (syst AB,J=8,5Hz, 2H); 7,31 (syst AB,J=8,5Hz, 2H); 7,36 (s, 1H); 7,40 (s, 1H) ($CDCl_3$) |
| 21a | H | H | H | H | H | N | CH | Cl | CH | 3 | huile | 2948, 2923, 2811, 2774, 1446, 1382, 1316, 971, 748, 695 film | 2,08 (quin,J=7,0Hz, 2H); 2,42 (t,J=7,0Hz, 2H); 2,58 (m, 2H); 2,67 (t,J=5,6Hz, 2H); 3,13 (m, 2H); 4,17 (t,J=6,9Hz, 2H); 6,07 (m, 1H); 7,23-7,45 (a.c., 7H) ($CDCl_3$) |
| 22a | H | H | H | H | H | $CCH_3$ | N | Cl | CCl | 3 | huile | 2923, 1533, 1405, 1379, 1246, 749 film | 1,95 (quin,J=7,2Hz, 2H); 2,39 (s, 3H); 2,46 (t,J=7,0Hz, 2H); 2,58 (m, 2H); 2,69 (t,J=4,9Hz, 2H); 3,13 (m, 2H); 3,96 (t,J=7,3Hz, 2H); 6,07 (m, 1H); 7,20-7,41 (a.c., 5H) ($CDCl_3$) |
| 23a | H | H | H | H | H | CPh | N | H | CH | 4 | huile | 2940, 1496, 1474, 1445, 1379, 1275, 774, 698 film | 1,51 (m, 2H); 1,81 (m, 2H); 2,40 (t,J=7,4Hz, 2H); 2,56 (m, 2H); 2,63 (t,J=4,9Hz, 2H); 3,09 (m, 2H); 4,04 (t,J=7,2Hz, 2H); 6,03 (m,1H); 7,03 (m, 1H); 7,13 (m, 1H); 7,22-7,48 (a.c., 8H); 7,58 (m, 2H) ($CDCl_3$) |

TABLEAU II (suite)

| Ex | $R_1$ | $R_2$ | $R_3$ | $R_4$ | $R_5$ | $Z_1$ | $Z_2$ | $R_6$ | $Z_4$ | n | P.F. | IR $cm^{-1}$ | $^1H$-RMN (300 MHz),δ (solvant) |
|---|---|---|---|---|---|---|---|---|---|---|---|---|---|
| 24a | H | H | $CH_3$ | H | H | CH | N | CH=CH-CH=CH-C | | 4 | 90-91°C | 2939, 2915, 1500, 1461, 1377, 1365, 750 KBr | 1,59 (m, 2H); 1,95 (m, 2H); 2,32 (s, 3H); 2,46 (t,J=7,3Hz, 2H); 2,53 (m, 2H); 2,63 (t,J=5,5Hz, 2H); 3,08 (m, 2H); 4,20 (t,J=6,95Hz, 2H); 6,00 (s, 1H); 7,11 (d,J=7,8Hz, 2H); 7,27 (a.c., 4H); 7,40 (m, 1H); 7,80 (m, 1H); 7,89 (s, 1H) ($CDCl_3$) |
| 25a | H | H | H | H | H | CH | N | Ph | CPh | 4 | 100-101°C | 3130, 2939, 2770, 1600, 1506, 1443, 1259, 954, 780, 774, 750, 696, 649 KBr | 1,46 (quin,J=7,5Hz, 2H); 1,65 (quin,J=7,6Hz, 2H); 2,33 (t,J=7,3Hz, 2H); 2,53 (m, 2H); 2,60 (m, 2H); 3,05 (m, 2H); 3,84 (t,J=7,2Hz, 2H); 6,02 (m, 1H); 7,05-7,50 (a.c., 15H); 7,61 (s, 1H) ($CDCl_3$) |

| Ex | $R_1$ | $R_2$ | $R_3$ | $R_4$ | $R_5$ | $Z_1$ | $Z_2$ | $R_6$ | $Z_4$ | n | P.F. | IR $cm^{-1}$ | $^1H$-RMN (300 MHz),δ (solvant) |
|---|---|---|---|---|---|---|---|---|---|---|---|---|---|
| 26a | CH=CH-CH=CH | | H | H | H | N | CH | Cl | CH | 4 | huile | 3057, 3043, 2942, 2806, 2768, 1378, 1365, 971, 801, 778 film | 1,61 (quin,J=7,5Hz, 2H); 1,95 (quin,J=7,6Hz, 2H); 2,51-2,57 (a.c., 4H); 2,76 (t,J=5,6Hz, 2H); 3,20 (m, 2H); 4,14 (t,J=7,1Hz, 2H); 5,74 (m, 1H); 7,26-7,50 (a.c., 6H); 7,75 (d,J=8Hz, 1H); 7,84 (m, 1H); 8,02 (m, 1H); ($CDCl_3$) |
| 27a | H | CH=CH-CH=CH | | H | H | N | CH | Cl | CH | 4 | 95-96°C | 3111, 2920, 2806, 1374, 1326, 966, 826, 749, 612, KBr | 1,57 (m,2H); 1,92 (m, 2H); 2,48 (m, 2H); 2,71 (a.c, 4H); 3,18 (m, 2H); 4,11 (m, 2H); 6,22 (m, 1H); 7,38-7,50 (a.c., 4H); 7,61 (m, 1H); 7,75-7,84 (a.c., 4H) ($CDCl_3$) |

TABLEAU II (suite)

| Ex | $R_1$ | $R_2$ | $R_3$ | $R_4$ | $R_5$ | $Z_1$ | $Z_2$ | $R_6$ | $Z_4$ | n | Sel / P.F. | IR $cm^{-1}$ | $^1$H-RMN (300 MHz),δ,J=Hz (solvant) |
|---|---|---|---|---|---|---|---|---|---|---|---|---|---|
| 28a | H | H | F | H | H | CH | N | CH=CH-CH=CH-C | | 2 | 135-136°C | 3050, 2920, 2780, 2760, 1510, 1492, 1459, 1224, 1202, 1161, 771, 751 KBr | 2,54 (m, 2H); 2,74 (t,J=5,6Hz, 2H); 2,92 (t,J=6,7Hz, 2H); 3,24 (m, 2H); 4,35 (t,J=6,7Hz, 2H); 5,98 (m, 1H); 7,00 (t,J=8,7Hz, 2H); 7,26-7,40 (a.c., 4H); 7,42 (m, 1H); 7,81 (m, 1H); 8,01 (s, 1H) ($CDCl_3$) |
| 29a | H | H | H | H | H | CH | N | CH=CH-CH=CH-C | | 4 | HCl 177-178°C | 2940, 2488, 1500, 1420, 1390, 742 KBr | 1,70-1,90 (a.c., 4H); 2,78 (m, 2H); 3,17 (m, 2H); 3,20-3,50 (b.a., 2H); 3,79 (m, 2H); 4,30 (t,J=6,6Hz, 2H); 6,15 (s, 1H); 7,17-7,40 (a.c., 5H); 7,45 (d,J=7,3Hz, 2H); 7,65 (m, 2H); 8,35 (s, 1H) (DMSO-$d_6$) |
| 30a | H | H | F | H | H | CH | N | CH=CH-CH=CH-C | | 4 | 106-108°C | 2942, 1512, 1498, 1460, 1376, 1221, 756 KBr | 1,59 (quin,J=7,5Hz, 2H); 1,96 (quin,J=7,5Hz, 2H); 2,40-2,50 (a.c., 4H); 2,63 (t,J=5,5Hz, 2H); 3,09 (m, 2H); 4,21 (t,J=7,1Hz, 2H); 5,97 (m, 1H); 6,98 (t,J=8,1Hz, 2H); 7,20-735 (a.c., 4H); 7,40 (m, 1H); 7,80 (m, 1H); 7,89 (s, 1H) ($CDCl_3$) |
| 31a | H | H | F | H | H | CH | N | CH=CH-CH=CH-C | | 4 | HCl | 2930, 1600, 1510, 1275 KBr | 1,70-2,00 (a.c., 4H); 2,78 (m, 2H); 3,20 (m, 2H); 3,20-3,60 (b.a., 2H); 3,81 (m, 2H); 4,38 (t,J=6,6Hz, 2H); 6,13 (s, 1H); 7,19 (t,J=8,7Hz, 2H); 7,33 (m, 2H); 7,49 (m, 2H); 7,71 (d,J=7,8Hz, 1H); 7,77 (d,J=7,6Hz, 1H); 8,79 (s, 1H); 11,20 (b.a., 1H) (DMSO-$d_6$) |
| 32a | H | $CF_3$ | H | H | H | $CCH_3$ | N | Cl | CCl | 4 | HCl 205-206°C | 2930, 2490, 1330, 1243, 1164, 1119, 1076 KBr | 1,67 (m, 2H); 1,79 (m, 2H); 2,33 (s, 3H); 2,79 (m, 1H); 2,91 (m, 1H); 3,10-3,20 (a.c., 3H); 3,55 (m, 1H); 3,77 (m, 1H); 3,91-4,00 (a.c., 3H); 6,33 (s, 1H); 7,58-7,80 (a.c., 4H); 11,32 (b.a., 1H) (DMSO-$d_6$) |

TABLEAU II (suite)

| Ex | $R_1$ | $R_2$ | $R_3$ | $R_4$ | $R_5$ | $Z_1$ | $Z_2$ | $R_6$ | $Z_4$ | n | Sel / P.F. | IR $cm^{-1}$ | $^1H$-RMN (300 MHz),δ,J=Hz (solvant) |
|---|---|---|---|---|---|---|---|---|---|---|---|---|---|
| 33a | H | H | F | H | H | N | CH | Cl | CH | 4 | HCl 191-192°C | 2543, 1512, 1232, 967, 807 KBr | 1,71-1,85 (a.c., 4H); 2,68 (m, 1H); 2,86 (m, 1H); 3,10-3,20 (a.c., 3H); 3,55 (m, 1H); 3,72 (m, 1H); 3,90 (m, 1H); 4,12 (t,J=6,5Hz, 2H); 6,14 (s, 1H); 7,20 (t,J=8,7Hz, 2H); 7,40-7,55 (a.c., 3H); 8,06 (s, 1H); 11,20 (b.a., 1H) ($DMSO-d_6$) |
| 34a | H | H | H | H | H | N | CH | CH=CH-CH=CH-C | | 4 | HCl 193-194°C | 2931, 2566, 742 KBr | 1,80 (m, 2H); 1,91 (m, 2H); 2,67 (m, 1H); 2,88 (m, 1H); 3,10-3,20 (a.c., 3H); 3,52 (m, 1H); 3,71 (m, 1H); 3,90 (m, 1H); 4,46 (t,J=6,7Hz, 2H); 6,15 (s, 1H); 7,14 (t,J=7,5Hz, 1H); 7,25-7,41 (a.c., 4H); 7,46 (d,J=8,6Hz, 2H); 7,71 (d,J=8,6Hz, 1H); 7,75 (d,J=8,3Hz, 1H); 8,08 (s, 1H); 11,18 (b.a., 1H) ($DMSO-d_6$) |
| 35a | H | H | F | H | H | $CCH_3$ | N | Cl | CCl | 4 | HCl 160-161°C | 2930, 2590, 1512, 1409, 1241, 827 KBr | 1,67 (m, 2H); 1,79 (m, 2H); 2,33 (s, 3H); 2,67 (m, 1H); 2,90 (m, 1H); 3,10-3,25 (a.c., 3H); 3,54 (m, 1H); 3,72 (m, 1H); 3,85-3,98 (a.c., 3H); 6,13 (s, 1H); 7,19 (m, 2H); 7,50 (m, 2H); 11,28 (b.a., 1H) ($DMSO-d_6$) |
| 36a | H | H | H | H | H | N | CH | 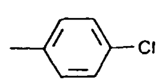 | CH | 4 | HCl 198-199°C | 2472, 1560, 1450, 1095, 955, 810, 745 KBr | 1,77 (m, 2H); 1,87 (m, 2H); 2,70 (m, 1H); 2,86 (m, 1H); 3,16 (a.c., 3H); 3,55 (m, 1H); 3,73 (m, 1H); 3,90 (m, 1H); 4,17 (t,J=6,6Hz, 2H); 6,15 (m, 1H); 7,25-7,47 (a.c., 7H); 7,59 (m, 2H); 7,90 (s, 1H); 8,27 (s, 1H); 10,91 (b.a., 1H) ($DMSO-d_6$) |
| 37a | H | H | H | H | H | N | CH | 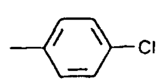 | CH | 4 | 126-127°C | 2935, 1570, 1493, 1455, 1379, 1091, 953, 815, 746 KBr | 1,60 (m, 2H); 1,97 (m, 2H); 2,48 (t,J=7,3Hz, 2H); 2,56 (m, 2H); 2,67 (t,J=5,1Hz, 2H); 3,13 (m, 2H); 4,18 (t,J=7,1Hz, 2H); 6,05 (m, 1H); 7,23-7,40 (a.c., 9H); 7,61 (s, 1H); 7,74 (s, 1H) ($CDCl_3$) |

TABLEAU II (suite)

| Ex | $R_1$ | $R_2$ | $R_3$ | $R_4$ | $R_5$ | $Z_1$ | $Z_2$ | $R_6$ | $Z_4$ | n | Sel / P.F. | IR $cm^{-1}$ | $^1H$-RMN (300 MHz),δ,J=Hz (solvant) |
|---|---|---|---|---|---|---|---|---|---|---|---|---|---|
| 38a | H | H | F | H | H | N | CH | H | N | 4 | HCl 166-168°C | 3450, 2429, 2707, 2593, 1512, 1437, 1230, 816, 626 KBr | 1,74 (m, 2H); 1,86 (m, 2H); 2,68 (m, 1H); 2,84 (m, 1H); 3,16 (a.c., 3H); 3,53 (m, 1H); 3,70 (m, 1H); 3,91 (m, 1H); 4,27 (t,J=6,7Hz, 2H); 6,12 (s, 1H); 7,19 (t,J=8,9Hz, 2H); 7,50 (dd,J=7,2Hz, J'=5,5Hz, 2H); 8,23 (s, 1H); 8,93 (s, 1H); 11,02 (b.a., 1H) (DMSO-$d_6$) |
| 39a | H | H | F | H | H | N | CH | H | N | 4 | Huile | 2944, 2808, 2773, 1602, 1510, 1273, 1227, 1161, 1140, 846, 824, 681 film | 1,60 (m, 2H); 1,97 (m, 2H); 2,40-2,70 (a.c., 6H); 3,12 (m, 2H); 4,22 (t,J=6,9Hz, 2H); 5,99 (m, 1H); 6,98 (m, 2H); 7,35 (m, 2H); 7,95 (s, 1H); 8,07 (s,1H) ($CDCl_3$) |
| 40a | H | H | F | H | H | $CCH_3$ | N | CH=CH-CH=CH-C | | 4 | Huile | 2932, 1512, 1456, 1404, 1231, 744 film | 1,63 (m, 2H); 1,88 (m, 2H); 2,42-2,55 (a.c., 4H); 2,61 (s,3H); 2,65 (t,J=5,5Hz, 2H); 3,09 (m, 2H); 4,14 (t,J=7,3Hz, 2H); 5,97 (m, 1H); 6,99 (m, 2H); 7,19-7,35 (a.c., 5H); 7,68 (m, 1H) ($CDCl_3$) |
| 41a | H | H | F | H | H | N | CH | CH=CH-CH=CH-C | | 4 | Huile | 2932, 2805, 1511, 1465, 1230, 1160, 825, 752, 741 film | 1,57 (m, 2H); 1,99 (m, 2H); 2,42-2,50 (a.c., 4H); 2,62 (t,J=5,6Hz, 2H); 3,06 (m, 2H); 4,42 (t,J=6,9Hz, 2H); 5,95 (m, 1H); 6,97 (t,J=8,8Hz, 2H); 7,12 (m, 1H); 7,25-7,41 (a.c., 4H); 7,71 (d,J=8Hz, 1H); 7,99 (s, 1H) ($CDCl_3$) |
| 42a | H | H | F | H | H | N | | C-CH=CH-CH=CH | CH | 4 | 102-103°C | 2941, 1510, 1374, 1226, 1162, 806, 759, 741 KBr | 1,59 (quin., J=7,0Hz, 2H); 2,09 (quin., J=7,5Hz, 2H); 2,40-2,50 (a.c., 4H); 2,64 (t,J=6,2Hz, 2H); 3,10 (m, 2H); 4,45 (t,J=7,1Hz, 2H); 5,96 (m, 1H); 6,98 (t,J=8,8Hz, 2H); 7,07 (t,J=7,6Hz, 1H); 7,20-7,35 (a.c., 3H); 7,63 (d,J=8,5Hz, 1H); 7,71 (d,J=8,6Hz, 1H); 7,90 (s,1H) ($CDCl_3$) |

TABLEAU II (suite)

| Ex | $R_1$ | $R_2$ | $R_3$ | $R_4$ | $R_5$ | $Z_1$ | $Z_2$ | $R_6$ | $Z_4$ | n | Sel / P.F. | IR $cm^{-1}$ | $^1H$-RMN (300 MHz),δ,J=Hz (solvant) |
|---|---|---|---|---|---|---|---|---|---|---|---|---|---|
| 43a | H | H | F | H | H | N | C-CH=CH-CH=CH | | N | 4 | HCl 208-209°C | 2574, 2482, 1510, 1231, 745 KBr | 1,80 (m, 2H); 2,11 (quin., J=7,2Hz, 2H); 2,69 (m, 1H); 2,83 (m, 1H); 3,10-3,20 (a.c., 3H); 3,52 (m, 1H); 3,71 (m, 1H); 3,88 (m, 1H); 4,80 (t,J=6,3Hz, 2H); 6,11 (s, 1H); 7,19 (m, 2H); 7,41 (m, 2H); 7,50 (m, 2H); 7,91 (m, 2H); 11,07 (b.a., 1H) (DMSO-$d_6$) |
| 44a | H | H | F | H | H | N | C-CH=CH-CH=CH | | N | 4 | 76-77°C | 2913, 1511, 1470, 1380, 1327, 1224, 1172, 1132, 851, 826, 757 KBr | 1,60 (quin., J=7,5Hz, 2H); 2,19 (quin., J=8,2Hz, 2H); 2,41-2,59 (a.c., 4H); 2,64 (t,J=5,7Hz, 2H); 3,08 (m, 2H); 4,77 (t,J=7,0Hz, 2H); 5,95 (m, 1H); 6,97 (t,J=8,8Hz, 2H); 7,25-7,40 (a.c., 4H); 7,85 (m, 2H) ($CDCl_3$) |
| 45a | H | H | F | H | H | N | N | CH=CH-CH=CH-C | | 4 | HCl 204-205°C | 2928, 2680, 2573, 2559, 1515, 1454, 1272, 1242, 1224, 1166, 819, 745 KBr | 1,81 (m, 2H); 1,99 (m, 2H); 2,67 (m, 1H); 2,84 (m, 1H); 3,10-3,20 (a.c., 3H); 3,53 (m, 1H); 3,72 (m, 1H); 3,90 (m, 1H); 4,76 (t,J=6,9Hz, 2H); 6,12 (s,1H); 7,19 (t,J=8,8Hz, 2H); 7,39 (t,J=7,6 Hz, 1H); 7.45-7,60 (a.c., 3H); 7,94 (d,J=8,3Hz, 2H); 8,03 (d,J=8,3Hz, 2H); 11,04 (b.a., 1H) (DMSO-$d_6$) |
| 46a | H | H | F | H | H | N | N | CH=CH-CH=CH-C | | 4 | 88-90°C | 2939, 1510, 1229, 1209, 1164, 744 KBr | 1,58 (quin., J=7,5Hz, 2H); 2,07 (quin, J=7,5Hz, 2H); 2,40-2,50 (a.c., 4H); 2,61 (m, 2H); 3,05 (m, 2H); 4,66 (t,J=7,0Hz, 2H); 5,95 (m, 1H); 6,96 (t,J=8,8Hz, 2H); 7,23-7,38 (a.c., 3H); 7,44 (m, 1H); 7,52 (m, 1H); 8,04 (d,J=8,3Hz, 1H) ($CDCl_3$) |
| 47a | H | H | Cl | H | H | N | CH | Cl | CH | 4 | HCl 172-173°C | 3068, 2948, 1491, 1445, 1320, 1308, 1096, 968, 809, 799 KBr | 1,71 (m, 2H); 1,80 (m, 2H); 2,70 (m, 1H); 2,83 (m, 1H); 3,15-330 (a.c., 3H); 3,44 (m, 1H); 3,72 (m, 1H); 3,89 (m, 1H); 4,11 (t,J=6,5Hz, 2H); 6,20 (s, 1H); 7,41 (Syst. AB,$J_{AB}$=8,8Hz, 2H); 7,48 (Syst. AB,$J_{AB}$=8,8Hz, 2H); 7,52 (s, 1H); 8,04 (s, 1H); 10,98 (b.a., 1H) (DMSO-$d_6$) |

TABLEAU II (suite)

| Ex | $R_1$ | $R_2$ | $R_3$ | $R_4$ | $R_5$ | $Z_1$ | $Z_2$ | $R_6$ | $Z_4$ | n | Sel / P.F. | IR $cm^{-1}$ | $^1H$-RMN (300 MHz),δ,J=Hz (solvant) |
|---|---|---|---|---|---|---|---|---|---|---|---|---|---|
| 48a | H | H | H | H | H | N | CH | H | CH | 4 | HCl 180-181°C | 2955, 2929, 2530, 1445, 965, 761, 745 KBr | 1,70-190 (a.c., 4H); 2,69 (m, 1H); 2,89 (m, 1H); 3,10-3,20 (a.c., 3H); 3,53 (m, 1H); 3,70 (m, 1H); 3,91 (m, 1H); 4,15 (t,J=6,5Hz, 2H); 6,16 (m, 1H); 6,23 (m, 1H); 7,28-7,50 (a.c., 6H); 7,78 (m, 1H); 11,26 (b.a., 1H) (DMSO-$d_6$) |
| 49a | H | H | H | H | H | CH | N | H | N | 4 | HCl 122-123°C | 2937, 2370, 1503, 1276, 1142, 774, 755 KBr | 1,74 (m, 2H); 1,84 (m, 2H); 2,72 (m, 1H); 2,87 (m, 1H); 3,10-3,20 (a.c., 3H); 3,54 (m, 1H); 3,73 (m, 1H); 3,88 (m, 1H); 4,22 (t,J=6,6Hz, 2H); 6,15 (s, 1H); 7,27-7,70 (a.c., 3H); 7,47 (m, 2H); 7,97 (s, 1H); 8,59 (s, 1H); 11,20 (b.a., 1H) (DMSO-$d_6$) |
| 50a | H | H | H | H | H | CPh | N | H | CH | 4 | HCl 170-171°C | 2930, 2554, 1469, 1459, 1444, 1278, 1075, 774, 762, 749, 732, 711, 702, 690 KBr | 1,62-1,78 (a.c., 4H); 2,75 (m, 2H); 3,00 (m, 2H); 3,25 (m, 2H); 3,69 (m, 2H); 4,08 (t,J=6,7Hz, 2H); 6,13 (s, 1H); 7,07 (s, 1H); 7,24-7,40 (a.c., 3H); 7,42-7,52 (a.c., 6H); 7,62 (Syst. AB,$J_{AB}$=7,6Hz, 2H) (DMSO-$d_6$) |
| 51a | H | H | H | H | H | CH | CH | H | CH | 4 | HCl 197-199°C | 2930, 2482, 1448, 1280, 1090, 732 KBr | 1,60-1,80 (a.c., 4H); 2,70 (m, 1H); 2,84 (m, 1H); 3,08-3,22 (a.c., 3H); 3,50 (m, 1H); 3,71 (m, 1H); 3,86-3,96 (a.c., 3H); 5,97 (t,J=2,1Hz, 2H); 6,16 (m, 1H); 6,76 (t,J=2,1Hz, 2H); 7,25-7,50 (a.c., 5H); 10,74 (b.a., 1H) (DMSO-$d_6$) |
| 52a | H | H | H | H | H | CH | CH | H | CH | 4 | 58-60°C | 2928, 1498, 1280, 1262, 1137, 1087, 1060, 747, 723, 691 KBr | 1,58 (m, 2H); 1,84 (m, 2H); 2,47 (t,J=7,5Hz, 2H); 2,58 (m, 2H); 2,68 (m, 2H); 3,13 (m, 2H); 3,92 (t,J=7,1Hz, 2H); 6,06 (m, 1H); 6,15 (t,J=2,2Hz, 2H); 6,67 (t,J=2,2Hz, 2H); 7,24-7,42 (a.c., 5H) ($CDCl_3$) |

TABLEAU II (suite)

| Ex | $R_1$ | $R_2$ | $R_3$ | $R_4$ | $R_5$ | $Z_1$ | $Z_2$ | $R_6$ | $Z_4$ | n | Sel / P.F. | IR $cm^{-1}$ | $^1H$-RMN (300 MHz),δ,J=Hz (solvant) |
|---|---|---|---|---|---|---|---|---|---|---|---|---|---|
| 53a | H | H | H | H | H | N | CCl | CH=CH-CH=CH-C | | 4 | Huile | 2939, 1495, 1467, 1338, 745 film | 1,58 (quin, J=7,6Hz, 2H); 1,99 (quin, J=7,6Hz, 2H); 2,47 (m, 2H); 2,55 (m, 2H); 2,65 (m, 2H); 3,10 (m, 2H); 4,36 (t,J=7,1Hz, 2H); 6,04 (m, 1H); 7,18-7,42 (a.c., 8H); 7,67 (d,J=7,6Hz, 1H) ($CDCl_3$) |
| 54a | H | H | H | H | H | N | CCl | CH=CH-CH=CH-C | | 4 | HCl 164-165°C | 3460, 2940, 2550, 1338, 743 KBr | 1,80 (m, 2H); 1,90 (m, 2H); 2,70 (m, 1H); 2,87 (m, 1H); 3,07-3,22 (a.c., 3H); 3,52 (m, 1H); 3,71 (m, 1H); 3,87 (m, 1H); 4,43 (t,J=6,6Hz, 2H); 6,14 (s, 1H); 7,20-7,52 (a.c., 7H); 7,65 (m, 1H); 7,79 (m, 1H); 11,16 (b.a., 1H) (DMSO-$d_6$) |

## ESSAIS BIOLOGIQUES

LIAISON AU RECEPTEUR DE SEROTONINE ($5HT_{1A}$)

On utilise un homogénéisat d'hippocampe de rat en suivant une modification du procédé de S.J. Peroutka, J. of Neurochem., 47(2), 529-540 (1986). Comme radiocoordinat on utilise [3H]8-OH-DPAT et pour mesurer l'union non spécifique on utilise de la sérotonine. Le temps d'incubation est de 15 minutes à une température de 37°C. On sépare le radiocoordinat uni à la protéine par filtration sur filtres de fibre de verre et on détermine la radioactivité retenue sur le filtre par scintillation liquide. On calcule les constantes d'inhibition (Ki, nM) par analyse de régression non linéaire en utilisant le programme EBDA/LIGAND (Munson et Rodbard, Analytical Biochemistry, 107, 220 (1980).

LIAISON AU RECEPTEUR SIGMA

On utilise un homogénéisat de cerveau (moins le cervelet) de cobaye en suivant une modification du procédé de L. Radesca et al., J. Med. Chem., 34, 3058-3065 (1991). Comme radiocoordinat on utilise [$^3$H] (+)-3-PPP et pour mesurer l'union non spécifique on utilise de l'halopéridol. Le temps d'incubation est de 120 minutes à une température de 25°C. On sépare le radiocoordinat uni à la protéine par filtration sur filtres de fibre de verre et on détermine la radioactivité retenue sur le filtre par scintillation liquide. On calcule les constantes d'inhibition (Ki, nM) par analyse de régression non linéaire en utilisant le programme EBDA/LIGAND (Munson et Rodbard, Analytical Biochemistry, 107, 220 (1980).

TABLEAU III

| LIAISON | | | | |
|---|---|---|---|---|
| | Sigma | | $5\text{-}HT_{1A}$ | |
| Exemple | Ki (nM) | %Des ($10_{-6}$M) | Ki (nM) | %Des ($10_{-6}$M) |
| 1 | 6,6 | 93 | | |
| 2 | 15 | 90 | | |
| 5 | 6,4 | 94 | | 12 |
| 6 | 4,3 | 95 | | |
| 1a | 46 | 86 | 37 | 94 |
| 2a | | 82 | | 78 |
| 3a | 11 | 97 | 56 | 90 |
| 4a | 11 | 93 | 199 | 72 |
| 5a | 19 | 96 | 6,1 | 97 |
| 6a | | 91 | 9,0 | 100 |
| 7a | | 96 | 0,4 | 98 |
| 8a | | 97 | | 90 |
| 9a | | 97 | 4,6 | 100 |
| 10a | 40 | 88 | 1,3 | 100 |
| 11a | | 92 | | 93 |
| 14a | | 94 | | 94 |
| 15a | 8,4 | 94 | | 98 |
| 16a | 17 | 95 | | 86 |
| BMY 14802 | 733 | | | |

La posologie quotidienne en médecine humaine est comprise entre 1 milligramme et 500 milligrammes de produit qui peut être administré en une ou plusieurs prises. On prépare les compositions sous des formules compatibles avec le mode d'administration utilisé, comme par exemple comprimés, dragées, capsules, suppositoires, solutions ou suspensions. On prépare ces compositions par des procédés connus, et elles comprennent de 1 à 60 % en poids de principe actif (composé de formule générale I) et de 40 à 99 % en poids de véhicule pharmaceutique approprié et compatible avec le principe actif et la forme physique de la composition utilisée. A titre d'exemple on présente la formule d'un comprimé qui contient un produit de l'invention.

| Exemple de formule pour comprimé | |
|---|---|
| Exemple 11a | 5 mg |
| Lactose | 60 mg |
| Cellulose cristalline | 25 mg |
| Povidone K 90 | 5 mg |
| Amidon prégélatinisé | 3 mg |
| Dioxyde de silice colloïdal | 1 mg |
| Stéarate de magnésium | 1 mg |
| Poids total | 100 mg |

**Revendications**

1. Les composés de formule générale (1)

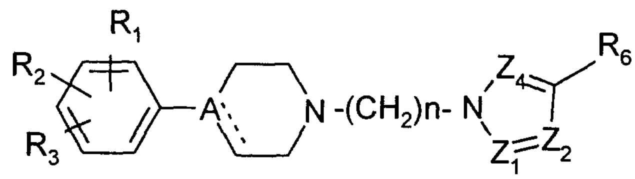

et leurs sels physiologiquement acceptables, dans laquelle

$R_1$, $R_2$ et $R_3$, identiques ou différents, représentent chacun un atome d'hydrogène, un atome d'halogène, un radical alkyle linéaire ou ramifié comprenant 1 à 6 atomes de carbone, un radical perfluoroalkyle, un radical phényle éventuellement substitué par un halogène ou un radical alkyle linéaire ou ramifié comprenant 1 à 6 atomes de carbone ou un radical alcoxyle, deux radicaux adjacents pouvant former un cycle aromatique ou saturé :

A représente un atome de carbone et la ligne pointillée représente une liaison additionnelle :

n vaut 4 ;

$Z_1$ représente un atome d'azote ou un atome de carbone substitué qui peut être représenté par C-$R_4$ ;
$Z_2$ représente un atome d'azote ou un atome de carbone substitué qui peut être représenté par C-$R_5$ ;
$Z_4$ représente un atome d'azote ou un atome de carbone substitué qui peut être représenté par C-$R_7$ ;
$R_4$, $R_5$, $R_6$ et $R_7$, identiques ou différents, représentent un atome d'hydrogène, un atome d'halogène, un radical alkyle linéaire ou ramifié comprenant 1 à 6 atomes de carbone, un radical hydroxyle, un radical alcoxyle, un radical phényle éventuellement substitué par un halogène ou un radical alkyle linéaire ou ramifié comprenant 1 à 6 atomes de carbone, ou bien deux radicaux adjacents peuvent faire partie d'un autre cycle, aromatique ou non,

le groupement

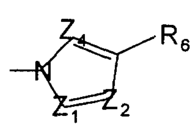

ne contenant pas de fonction carbonyle et ne représentant pas un radical tricyclique.

2. Les composés inclus dans la formule générale (I) selon la revendication 1, choisis dans le groupe suivant :

1. 4-chloro-1-[4-(4-hydroxy-4-phényl-1-pipéridinyl)butyl]-1H-pyrazole

2. 4,5-dichloro-1-[4-(4-hydroxy-4-phényl-1-pipéridinyl)butyl]-2-méthyl-1H-imidazole
3. 1-[4-(4-hydroxy-4-phényl-1-pipéridinyl)butyl]-1H-benzimidazole
4. 1-[4-hydroxy-4-phényl-1-pipéridinyl)butyl]-1H-1,2,4-triazole
5. 4-chloro-1-{4-[4-(4-chlorophényl)-4-hydroxy-1-pipéridinyl]butyl}-1H-pyrazole
6. 4,5-dichloro-1-{4-[4-hydroxy-4-(4-chlorophényl)-1-pipéridinyl]butyl}-2-méthyl-1H-imidazole
7. 4-chloro-1-{4-[4-hydroxy-4-(3-trifluorométhylphényl)-1-pipéridinyl]butyl}-1H-pyrazole
8. 4,5-dichloro-1-{4-[4-hydroxy-4-(3-trifluorométhylphényl)-1-pipéridinyl]butyl}-2-méthyl-1H-imidazole
9. 4,5-dichloro-1-{4-[4-(4-fluorophényl)-4-hydroxy-1-pipéridinyl]butyl}-2-méthyl-1H-imidazole
10. 1-[4-(4-hydroxy-4-phényl-1-pipéridinyl)butyl}indole
11. 4,5-dichloro-1-{4-[4-hydroxy-4-(4-méthylphényl)-1-pipéridinyl]butyl}-2-méthyl-1H-imidazole
12. 1-[4-(4-hydroxy-4-phényl-1-pipéridinyl)butyl]-1H-pyrazole
13. 1-[4-(4-hydroxy-4-phényl-1-pipéridinyl)butyl]-1H-indazole
14. 2-[4-(4-hydroxy-4-phényl-1-pipéridinyl)butyl]-2H-indazole
15. 4-chloro-1-{4-[4-hydroxy-4-(4-méthylphényl)-1-pipéridinyl]butyl}-1H-pyrazole
16. 4-chloro-1-{4-[4-hydroxy-4-(4-méthoxyphényl)-1-pipéridinyl]butyl}-1H-pyrazole
17. 1-[4-(4-hydroxy-4-phényl-1-pipéridinyl)butyl]-2-phényl-1H-imidazole
18. 1-{4-[4-hydroxy-4-(4-méthylphényl)-1-pipéridinyl]butyl}-1H-benzimidazole
19. 4,5-diphényl-1-[4-(4-hydroxy-4-phényl-1-pipéridinyl)butyl]-1H-imidazole
20. 4-chloro-1-{4-[4-hydroxy-4-(1-naphtyl)-1-pipéridinyl]butyl}-1H-pyrazole
21. 4-chloro-1-{4-[4-hydroxy-4-(2-naphthyl)-1-pipéridinyl]butyl}-1H-pyrazole
22. 4-chloro-1-{4-[4-phényl-1-(1,2,3,6-tétrahydropyridinyl)]butyl}-1H-pyrazole
23. 1-{4-[4-phényl-1-(1,2,3,6-tétrahydropyridinyl)]butyl}-1H-benzimidazole
24. 1-{4-[4-phényl-1-(1,2,3,6-tétrahydropyridinyl)]butyl}-1H-1,2,4-triazole
25. 4-chloro-1-{4-[4-(4-chlorophényl)-1-(1,2,3,6-tétrahydropyridinyl)]butyl}-1H-pyrazole
26. 4,5-dichloro-1-{4-[4-(4-chlorophényl)-1-(1,2,3,6-tétrahydropyridinyl)]butyl}-2-méthyl-1H-imidazole
27. 4-chloro-1-{4-[4-(3-trifluorométhylphényl)-1-(1,2,3,6-tétrahydropyridinyl)]butyl}-1H-pyrazole
28. 4,5-dichloro-2-méthyl-1-{4-[4-(3-trifluorométhylphényl)-1-(1,2,3,6-tétrahydropyridinyl)]butyl}-1H-imidazole
29. 4-chloro-1-{4-[4-(4-fluorophényl)-1-(1,2,3,6-tétrahydropyridinyl)]butyl}-1H-pyrazole
30. 4,5-dichloro-1-{4-[4-(4-fluorophényl)-1-(1,2,3,6-tétrahydropyridinyl)]butyl}-2-méthyl-1H-imidazole
31. 4,5-dichloro-1-{4-[4-phényl-1-(1,2,3,6-tétrahydropyridinyl)]butyl}-2-méthyl-1H-imidazole
32. Chlorhydrate de 4,5-dichloro-1-{4-[4-phényl-1-(1,2,3,6-tétrahydropyridinyl)]butyl}-2-méthyl-1H-imidazole
33. Dichlorhydrate de 4,5-dichloro-1-{4-[4-phényl-1-(1,2,3,6-tétrahydropyridinyl)]butyl}-2-méthyl-1H-imidazole
34. 1-{4-[4-(4-fluorophényl)-1-(1,2,3,6-tétrahydropyridinyl)]butyl}indole
35. 1-{4-[4-phényl-1-(1,2,3,6-tétrahydropyridinyl)]butyl}indole
36. 4,5-dichloro-2-méthyl-1-{4-[4-(4-méthylphényl)-1-(1,2,3,6-tétrahydropyridinyl)]butyl}-1H-imidazole
37. 1-{4-[4-phényl-1-(1,2,3,6-tétrahydropyridinyl)]butyl}-1H-pyrazole
38. 1-{4-[4-phényl-1-(1,2,3,6-tétrahydropyridinyl)]butyl}-1H-indazole
39. 2-{4-[4-phényl-1-(1,2,3,6-tétrahydropyridinyl)]buyl}-2H-indazole
40. 4-chloro-1-{4-[4-(4-méthylphényl)-1-(1,2,3,6-tétrahydropyridinyl)]butyl}-1H-pyrazole
41. 4-chloro-1-{4-[4-(4-méthoxyphényl)-1-(1,2,3,6-tétrahydropyridinyl)]butyl}-1H-pyrazole
42. 2-phényl-1-{4-[4-phényl-1-(1,2,3,6-tétrahydropyridinyl)]butyl}-1H-imidazole
43. 1-{4-[4-(4-méthylphényl)-1-(1,2,3,6-tétrahydropyridinyl)]butyl}-1H-benzimidazole
44. $R_4$, $R_5$, $R_6$ et $R_7$ ne font pas partie d'un autre cycle aromatique ou non, 4,5-diphényl-1-{4-[4-phényl-1-(1,2,3,6-tétrahydropyridinyl)]butyl}-1H-imidazole
45. 4-chloro-1-{4-[4-(1-naphtyl)-1-(1,2,3,6-tétrahydropyridinyl]butyl}-1H-pyrazole
46. 4-chloro-1-{4-[4-(2-naphtyl)-1-(1,2,3,6-tétrahydropyridinyl]butyl}-1H-pyrazole
47. Chlorhydrate de 1-{4-[4-phényl-1-(1,2,3,6-tétrahydropyridinyl)]butyl}-1 H-benzimidazole
48. 1-{4-[4-(4-fluorophényl)-1-(1,2,3,6-tétrahydropyridinyl)]butyl}-1H-benzimidazole
49. Chlorhydrate de 1-{4-[4-(4-fluorophényl)-1-(1,2,3,6-tétrahydropyridinyl)]butyl}-1H-benzimidazole
50. Chlorhydrate de 4,5-dichloro-2-méthyl-1-{4[4-(3-trifluorométhylphényl)1-(1,2,3,6-tétrahydropyridinyl)butyl}-1H-imidazole
51. Chlorhydrate de 4-chloro-1-{4-[4-(4-fluorophényl)-1-(1,2,3,6-tétrahydropyridinyl)]butyl}-1H-pyrazole
52. Chlorhydrate de 1-{4-[4-(4-phényl-1-(1,2,3,6,-tétrahydropyridinyl)]butyl}-1H-indazole
53. Chlorhydrate de 4,5-dichloro-1-{4-[4-(4-fluorophényl)-1-(1,2,3,6-tétrahydropyridinyl)]-butyl}-2-méthyl-1H-imidazole
54. Chlorhydrate de 4-(4-chlorophényl)-1-{4-[4-phényl-1-(1,2,3,6-tétrahydropyridinyl)]-butyl}-1H-pyrazole

55. 4-(4-chlorophényl)-1-{4-[4-phényl-1-(1,2,3,6-tétrahydro-pyridinyl)]butyl}-1H-pyrazole
56. Chlorhydrate de 1-{4-[4-(4-fluorophényl)-1-(1,2,3,6-tétrahydropyridinyl)]butyl}-1H-triazole
57. 1-{4-[4-(4-fluorophényl)-1-(1,2,3,6-tétrahydropyridinyl)]butyl}-1H-triazole
58. 1-{4-[4-(4-fluorophényl)-1-(1,2,3,6-tétrahydropyridinyl)]butyl}-2-méthyl-1H-benzimidazole
59. 1-{4-[4-(4-fluorophényl)-1-(1,2,3,6-tétrahydropyridinyl)]butyl}-1H-indazole
60. 2-{4-[4-(4-fluorophényl)-1-(1,2,3,6-tétrahydropyridinyl)]butyl}-2H-indazole
61. Chlorhydrate de 2-{4-[4-(4-fluorophényl)-1-(1,2,3,6-tétrahydropyridinyl)]butyl}-2H-benzotriazole
62. 2-{4-[4-(4-fluorophényl)-1-(1,2,3,6-tétrahydropyridinyl)]butyl}-2H-benzotriazole
63. Chlorhydrate de 1-{4-[4-(4-fluorophényl)-1-(1,2,3,6-tétrahydropyridinyl)]butyl}-1H-benzotriazole
64. 1-{4-[4-(4-fluorophényl)-1-(1,2,3,6-tétrahydropyridinyl)]butyl}-1H-benzotriazole
65. Chlorhydrate de 4-chloro-1-{4-[4-(4-chlorophényl)-1-(1,2,3,6-tétrahydropyridinyl)]butyl}-1H-pyrazole
66. Chlorhydrate de 1-{4-[4-phényl-1-(1,2,3,6-tétrahydropyridinyl)]butyl}-1H-pyrazole
67. Chlorhydrate de 1-{4-[4-phényl-1-(1,2,3,6-tétrahydropyridinyl)]butyl}-1H-triazole
68. Chlorhydrate de 2-phényl-1-{4-[4-phényl-1-(1,2,3,6-tétrahydropyridinyl)]butyl-1H-imidazole
69. Chlorhydrate de 1-{4-[4-phényl-1-(1,2,3,6-tétrahydropyridinyl)]butyl}-1H-pyrrole
70. 1-{4-[4-phényl-1-(1,2,3,6-tétrahydropyridinyl)]butyl}-1H-pyrrole
71. 4-(4-chlorophényl)-1-[4-(4-hydroxy-4-phényl-1-pipéridinyl)butyl]1H-pyrazole
72. 1-{4-[4-(4-fluorophényl)-4-hydroxy-1-pipéridinyl]butyl}-1H-benzimidazole
73. 4-chloro-1-{4-[4-hydroxy-4-(3-trifluorométhylphényl)-1-pipéridinyl]butyl}-1H-pyrazole
74. 1-{4-[4-(4-fluorophényl)-4-hydroxy-1-pipéridinyl]butyl}-1H-indazole
75. 2-{4-[4-(4-fluorophényl)-4-hydroxy-1-pipéridinyl]butyl}-2H-indazole
76. 2-{4-[4-(4-fluorophényl)-4-hydroxy-1-pipéridinyl]butyl}-2H-benzotriazole
77. 1-{4-[4-(4-fluorophényl)-4-hydroxy-1-pipéridinyl]butyl}-2H-benzotriazole
78. 3-chloro-1-{4-[4-phényl-1-(1,2,3,6-tétrahydropyridinyl)butyl}-1H-indazole
79. Chlorhydrate de 3-chloro-1-{4-[4-phényl-1-(1,2,3,6-tétrahydropyridinyl)butyl}-1H-indazole.

**3.** Procédés de préparation des composés de formule générale (I) selon l'une des revendications 1 ou 2, **caractérisés en ce qu'**on effectue, au minimum, l'une des réactions suivantes :

3.1/ réaction d'un composé de spiranne de formule générale (II)

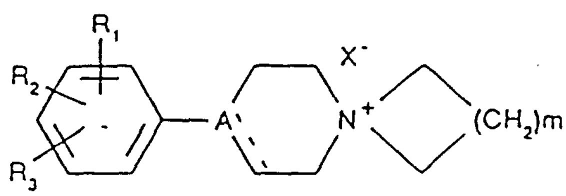

(II)

dans laquelle
$R_1$, $R_2$, $R_3$ et A ont la signification indiquée ci-dessus, m vaut 3 et X représente un groupe sortant, comme chloro, bromo, mésyloxy ou tosyloxy,
avec un hétérocycle azoté de formule générale (III)

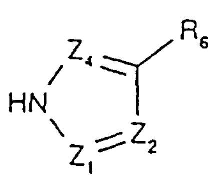

(III)

dans laquelle

$Z_1$, $Z_2$, $Z_4$ et $R_6$ ont la signification indiquée ci-dessus ;
3.2/ réaction simultanée "one pot" entre un dérivé de formule générale (IV), un agent alkylant de formule générale (V) et un hétérocycle azoté de formule générale (III)

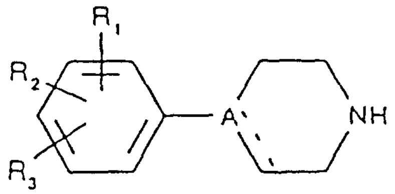
( IV )

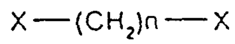
( V )

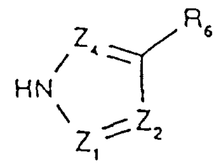
( III )

où
$R_1$, $R_2$, $R_3$, A, X, n, $Z_1$, $Z_2$, $Z_4$ et $R_6$ ont la signification indiquée ci-dessus ;
3.3/ réaction dans des conditions d'alkylation des amines de formule générale (IV)

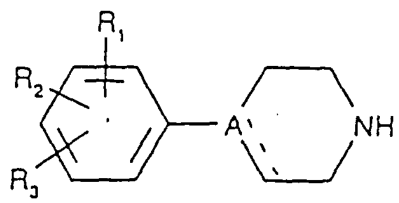
( IV )

dans laquelle
$R_1$, $R_2$, $R_3$ et A ont la signification indiquée ci-dessus, avec les dérivés de formule générale (VI)

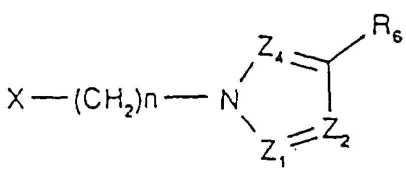
( VI )

dans laquelle
X, n, $Z_1$, $Z_2$, $Z_4$ et $R_6$ out la signification indiquée ci-dessus ;
3.4/ réaction dans des conditions d'alkylation d'un composé de formule générale (VII)

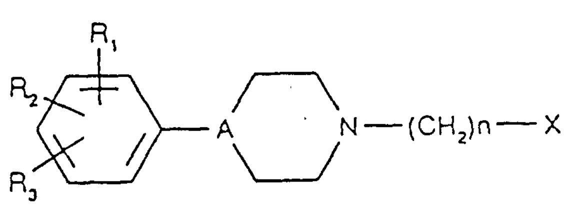
( VII )

dans laquelle
$R_1$, $R_2$, $R_3$, n, X et A ont la signification indiquée ci-dessus, avec un hétérocycle azoté de formule générale (III)

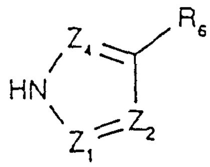

( III )

dans laquelle
$Z_1$, $Z_2$, $Z_4$ et $R_6$ ont la signification indiquée ci-dessus ;
3.5/ déshydratation de composés de formule générale (I)

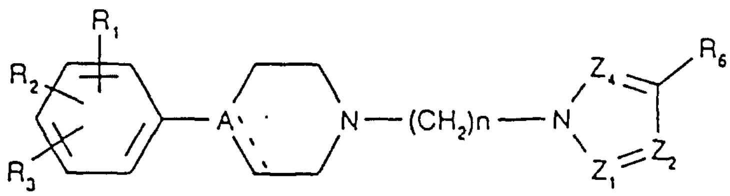

( I )

dans laquelle
$R_1$, $R_2$, $R_3$, n, $Z_1$, $Z_2$, $Z_4$ et $R_6$ ont la signification indiquée ci-dessus, A représente un atome de carbone uni à un groupe hydroxyle (C-OH) et la ligne pointillée représente un défaut de liaison additionnelle ;
3.6/ addition de réactifs organo-métalliques - par exemple phényl-lithium ou bromure de phényl-magnésium - à des composés de formule générale (VIII)

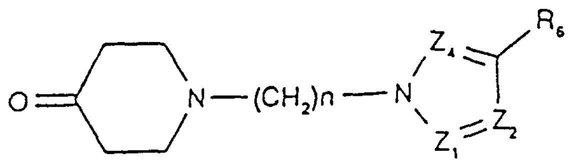

( VIII )

dans laquelle
n, $Z_1$, $Z_2$, $Z_4$ et $R_6$ ont la signification indiquée ci-dessus ;
3.7/ réduction de composés de formule générale (IX)

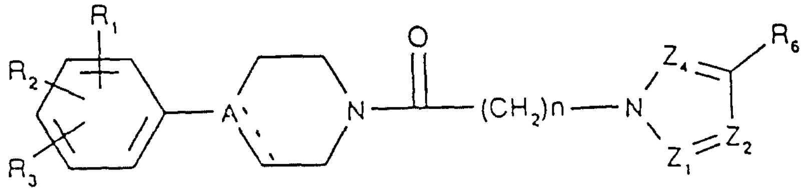

( IX )

dans laquelle
$R_1$, $R_2$, $R_3$, A, n, $Z_1$, $Z_2$, $Z_4$ et $R_6$ ont la signification indiquée ci-dessus.

**4.** Comme médicaments, les composés de formule générale (I) et leurs sels physiologiquement acceptables selon l'une des revendications 1 ou 2, en particulier comme médicaments à activité thérapeutique pour le traitement de l'anxiété, la psychose, l'épilepsie, la convulsion, les problèmes de motricité, l'amnésie, les maladies cérébrovasculaires, la démence sénile.

**5.** Compositions pharmaceutiques **caractérisées par le fait qu'**elles contiennent, outre un excipient pharmaceutiquement acceptable, au moins un composé de formule générale (I) ou un de ses sels physiologiquement acceptables, selon l'une des revendications 1 ou 2.

## Claims

**1.** Compounds of general formula (I)

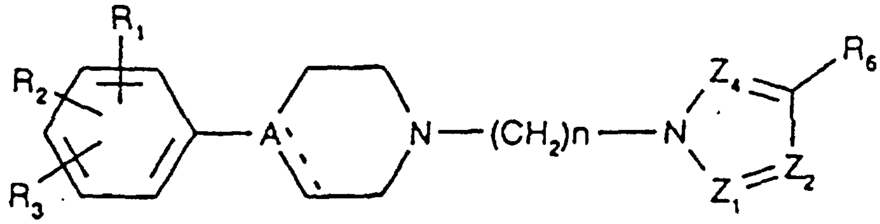

and their physiologically acceptable salts, in which $R_1$, $R_2$ and $R_3$, which are identical or different, each represent a hydrogen atom, a halogen atom, a linear or branched alkyl radical comprising 1 to 6 carbon atoms, a perfluoroalkyl radical, a phenyl radical optionally substituted by a halogen or a linear or branched alkyl radical comprising 1 to 6 carbon atoms or an alkoxyl radical, it being possible for two adjacent radicals to form an aromatic or saturated ring,

A represents a carbon atom and the dotted line represents an additional bond,
n has the value 4,
$Z_1$ represents a nitrogen atom or a substituted carbon atom which can be represented by C-$R_4$,
$Z_2$ represents a nitrogen atom or a substituted carbon atom which can be represented by C-$R_5$,
$Z_4$ represents a nitrogen atom or a substituted carbon atom which can be represented by C-$R_7$,
$R_4$, $R_5$, $R_6$ and $R_7$, which are identical or different, represent a hydrogen atom, a halogen atom, a linear or branched alkyl radical comprising 1 to 6 carbon atoms, a hydroxyl radical, an alkoxyl radical, a phenyl radical optionally substituted by a halogen or a linear or branched alkyl radical comprising 1 to 6 carbon atoms or else two adjacent radicals can form part of another ring, which may or may not be aromatic, the group

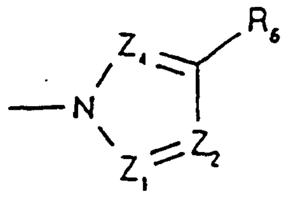

not comprising a carbonyl functional group and not representing a tricyclic radical.

**2.** Compounds included in the general formula (I) according to Claim 1, chosen from the following group:

1. 4-Chloro-1-[4-(4-hydroxy-4-phenyl-1-piperidinyl)-butyl]-1H-pyrazole
2. 4,5-Dichloro-1-[4-(4-hydroxy-4-phenyl-1-piperidinyl)butyl]-2-methyl-1H-imidazole
3. 1-[4-(4-Hydroxy-4-phenyl-1-piperidinyl)butyl]-1H-benzimidazole
4. 1-[4-Hydroxy-4-phenyl-1-piperidinyl)butyl]-1H-1,2,4-triazole
5. 4-Chloro-1-{4-[4-(4-chlorophenyl)-4-hydroxy-1-piperidinyl]butyl}-1H-pyrazole

6. 4,5-Dichloro-1-{4-[4-hydroxy-4-(4-chlorophenyl)-1-piperidinyl]butyl}-2-methyl-lH-imidazole
7. 4-Chloro-1-{4-[4-hydroxy-4-(3-trifluoromethylphenyl)-1-piperidinyl]butyl}-1H-pyrazole
8. 4,5-Dichloro-1-{4-[4-hydroxy-4-(3-trifluoromethylphenyl)-1-piperidinyl]butyl}-2-methyl-1H-imidazole
9. 4,5-Dichloro-1-{4-[4-(4-fluorophenyl)-4-hydroxy-1-piperidinyl]butyl}-2-methyl-lH-imidazole
10. 1-[4-(4-Hydroxy-4-phenyl-1-piperidinyl)butyl}indole
11. 4,5-Dichloro-1-{4-[4-hydroxy-4-(4-methylphenyl)-1-piperidinyl]butyl}-2-methyl-1H-imidazole
12. 1-[4-(4-Hydroxy-4-phenyl-1-piperidinyl)butyl]-1H-pyrazole
13. 1-[4-(4-Hydroxy-4-phenyl-1-piperidinyl)butyl]-1H-indazole
14. 2-[4-(4-Hydroxy-4-phenyl-1-piperidinyl)butyl]-2H-indazole
15. 4-Chloro-1-{4-[4-hydroxy-4-(4-methylphenyl)-1-piperidinyl]butyl}-1H-pyrazole
16. 4-Chloro-1-{4-[4-hydroxy-4-(4-methoxyphenyl)-1-piperidinyl]butyl}-1H-pyrazole
17. 1-[4-(4-Hydroxy-4-phenyl-1-piperidinyl)butyl]-2-phenyl-1H-imidazole
18. 1-{4-[4-Hydroxy-4-(4-methylphenyl)-1-piperidinyl]-butyl}-1H-benzimidazole
19. 4,5-Diphenyl-1-[4-(4-hydroxy-4-phenyl-1-piperidinyl)butyl]-1H-imidazole
20. 4-Chloro-1-{4-[4-hydroxy-4-(1-naphthyl)-1-piperidinyl]butyl}-1H-pyrazole
21. 4-Chloro-1-{4-[4-hydroxy-4-(2-naphthyl)-1-piperidinyl]butyl}-1H-pyrazole
22. 4-Chloro-1-{4-[4-phenyl-1-(1,2,3,6-tetrahydropyridinyl)]butyl}-1H-pyrazole
23. 1-{4-[4-Phenyl-1-(1,2,3,6-tetrahydropyridinyl)]butyl}-1H-benzimidazole
24. 1-{4-[4-Phenyl-1-(1,2,3,6-tetrahydropyridinyl)]butyl}-1H-1,2,4-triazole
25. 4-Chloro-1-{4-[4-(4-chlorophenyl)-1-(1,2,3,6-tetrahydropyridinyl)]butyl}-1H-pyrazole
26. 4,5-Dichloro-1-{4-[4-(4-chlorophenyl)-1-(1,2,3,6-tetrahydropyridinyl)]butyl}-2-methyl-1H-imidazole
27. 4-Chloro-1-{4-[4-(3-trifluoromethylphenyl)-1-(1,2,3,6-tetrahydropyridinyl)]butyl}-1H-pyrazole
28. 4,5-Dichloro-2-methyl-1-{4-[4-(3-trifluoromethylphenyl)-1-(1,2,3,6-tetrahydropyridinyl)]butyl}-1H-imidazole
29. 4-Chloro-1-{4-[4-(4-fluorophenyl)-1-(1,2,3,6-tetrahydropyridinyl)]butyl}-1H-pyrazole
30. 4,5-Dichloro-1-{4-[4-(4-fluorophenyl)-1-(1,2,3,6-tetrahydropyridinyl)]butyl}-2-methyl-1H-imidazole
31. 4,5-Dichloro-1-{4-[4-phenyl-1-(1,2,3,6-tetrahydropyridinyl)]butyl}-2-methyl-1H-imidazole
32. 4,5-Dichloro-1-{4-[4-phenyl-1-(1,2,3,6-tetrahydropyridinyl)]butyl}-2-methyl-1H-imidazole hydrochloride
33. 4,5-Dichloro-1-{4-[4-phenyl-1-(1,2,3,6-tetrahydropyridinyl)]butyl}-2-methyl-1H-imidazole dihydrochloride
34. 1-{4-[4-(4-Fluorophenyl)-1-(1,2,3,6-tetrahydropyridinyl)]butyl}indole
35. 1-{4-[4-Phenyl-1-(1,2,3,6-tetrahydropyridinyl)]-butyl}indole
36. 4,5-Dichloro-2-methyl-1-{4-[4-(4-methylphenyl)-1-(1,2,3,6-tetrahydropyridinyl)]butyl}-1H-imidazole
37. 1-{4-[4-Phenyl-1-(1,2,3,6-tetrahydropyridinyl)]-butyl}-1H-pyrazole
38. 1-{4-[4-Phenyl-1-(1,2,3,6-tetrahydropyridinyl)]-butyl}-1H-indazole
39. 2-{4-[4-Phenyl-1-(1,2,3,6-tetrahydropyridinyl)]-butyl}-2H-indazole
40. 4-Chloro-1-{4-[4-(4-methylphenyl)-1-(1,2,3,6-tetrahydropyridinyl)]butyl}-1H-pyrazole
41. 4-Chloro-1-{4-[4-(4-methoxyphenyl)-1-(1,2,3,6-tetrahydropyridinyl)]butyl}-1H-pyrazole
42. 2-Phenyl-1-{4-[4-phenyl-1-(1,2,3,6-tetrahydropyridinyl)]butyl}-1H-imidazole
43. 1-{4-[4-(4-Methylphenyl)-1-(1,2,3,6-tetrahydropyridinyl)]butyl}-1H-benzimidazole
44. $R_4$, $R_5$, $R_6$ and $R_7$ do not form part of another aromatic or non-aromatic ring, 4,5-diphenyl-1-{4-[4-phenyl-1-(1,2,3,6-tetrahydropyridinyl)]butyl}-1H-imidazole
45. 4-Chloro-1-{4-[4-(1-naphthyl)-1-(1,2,3,6-tetrahydropyridinyl]butyl}-1H-pyrazole
46. 4-Chloro-1-{4-[4-(2-naphthyl)-1-(1,2,3,6-tetrahydropyridinyl]butyl}-1H-pyrazole
47. 1-{4-[4-Phenyl-1-(1,2,3,6-tetrahydropyridinyl)]-butyl}-1H-benzimidazole hydrochloride
48. 1-{4-[4-(4-Fluorophenyl)-1-(1,2,3,6-tetrahydropyridinyl)]butyl}-1H-benzimidazole
49. 1-{4-[4-(4-Fluorophenyl)-1-(1,2,3,6-tetrahydropyridinyl)]butyl}-1H-benzimidazole hydrochloride
50. 4,5-Dichloro-2-methyl-1-{4[4-(3-trifluoromethylphenyl)1-(1,2,3,6-tetrahydropyridinyl)butyl}-1H-imidazole hydrochloride
51. 4-Chloro-1-(4-[4-(4-fluorophenyl)-1-(1,2,3,6-tetrahydropyridinyl)]butyl}-1H-pyrazole hydrochloride
52. 1-{4-[4-(4-Phenyl-1-(1,2,3,6-tetrahydropyridinyl)]-butyl}-1H-indazole hydrochloride
53. 4,5-Dichloro-1-{4-[4-(4-fluorophenyl)-1-(1,2,3,6-tetrahydropyridinyl)]butyl}-2-methyl-1H-imidazole hydrochloride
54. 4-(4-Chlorophenyl)-1-{4-[4-phenyl-1-(1,2,3,6-tetrahydropyridinyl)]butyl}-1H-pyrazole hydrochloride
55. 4-(4-Chlorophenyl)-1-{4-[4-phenyl-1-(1,2,3,6-tetrahydropyridinyl)]butyl}-1H-pyrazole
56. 1-{4-[4-(4-Fluorophenyl)-1-(1,2,3,6-tetrahydropyridinyl)]butyl}-1H-triazole hydrochloride
57. 1-{4-[4-(4-Fluorophenyl)-1-(1,2,3,6-tetrahydropyridinyl)]butyl}-1H-triazole
58. 1-(4-[4-(4-Fluorophenyl)-1-(1,2,3,6-tetrahydropyridinyl)]butyl)-2-methyl-1H-benzimidazole
59. 1-{4-[4-(4-Fluorophenyl)-1-(1,2,3,6-tetrahydropyridinyl)]butyl}-1H-indazole

60. 2-{4-[4-(4-Fluorophenyl)-1-(1,2,3,6-tetrahydropyridinyl)]butyl}-2H-indazole
61. 2-{4-[4-(4-Fluorophenyl)-1-(1,2,3,6-tetrahydropyridinyl)]butyl}-2H-benzotriazole hydrochloride
62. 2-{4-[4-(4-Fluorophenyl)-1-(1,2,3,6-tetrahydropyridinyl)]butyl}-2H-benzotriazole
63. 1-{4-[4-(4-Fluorophenyl)-1-(1,2,3,6-tetrahydropyridinyl)]butyl}-1H-benzotriazole hydrochloride
64. 1-{4-[4-(4-Fluorophenyl)-1-(1,2,3,6-tetrahydropyridinyl)]butyl}-1H-benzotriazole
65. 4-Chloro-1-{4-[4-(4-chlorophenyl)-1-(1,2,3,6-tetrahydropyridinyl)]butyl}-1H-pyrazole hydrochloride
66. 1-{4-[4-Phenyl-1-(1,2,3,6-tetrahydropyridinyl)]-butyl}-1H-pyrazole hydrochloride
67. 1-{4-[4-Phenyl-1-(1,2,3,6-tetrahydropyridinyl)]-butyl}-1H-triazole hydrochloride
68. 2-Phenyl-1-{4-[4-phenyl-1-(1,2,3,6-tetrahydropyridinyl)]butyl}-1H-imidazole hydrochloride
69. 1-{4-[4-Phenyl-1-(1,2,3,6-tetrahydropyridinyl)]-butyl}-1H-pyrrole hydrochloride
70. 1-{4-[4-Phenyl-1-(1,2,3,6-tetrahydropyridinyl)]-butyl}-1H-pyrrole
71. 4-(4-Chlorophenyl)-1-[4-(4-hydroxy-4-phenyl-1-piperidinyl)butyl]1H-pyrazole
72. 1-{4-[4-(4-Fluorophenyl)-4-hydroxy-1-piperidinyl]-butyl}-1H-benzimidazole
73. 4-Chloro-1-{4-[4-hydroxy-4-(3-trifluoromethylphenyl)-1-piperidinyl]butyl}-1H-pyrazole
74. 1-{4-[4-(4-Fluorophenyl)-4-hydroxy-1-piperidinyl]-butyl}-1H-indazole
75. 2-{4-[4-(4-Fluorophenyl)-4-hydroxy-1-piperidinyl]-butyl}-2H-indazole
76. 2-{4-[4-(4-Fluorophenyl)-4-hydroxy-1-piperidinyl]-butyl}-2H-benzotriazole
77. 1-{4-[4-(4-Fluorophenyl)-4-hydroxy-1-piperidinyl]-butyl}-2H-benzotriazole
78. 3-Chloro-1-{4-[4-phenyl-1-(1,2,3,6-tetrahydropyridinyl)butyl}-1H-indazole
79. 3-Chloro-1-{4-[4-phenyl-1-(1,2,3,6-tetrahydropyridinyl)butyl}-1H-indazole hydrochloride.

**3.** Processes for the preparation of the compounds of general formula (I) according to either of Claims 1 and 2, **characterized in that** at least one of the following reactions is carried out:

3.1/ reaction of a spiran compound of general formula (II)

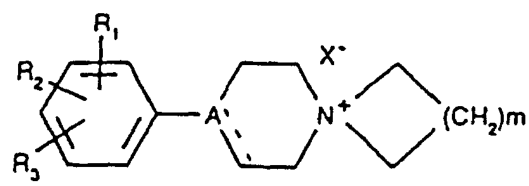

(II)

in which
$R_1$, $R_2$, $R_3$ and A have the meaning indicated above, m has the value 3 and X represents a leaving group, such as chloro, bromo, mesyloxy or tosyloxy,
with a nitrogenous heterocycle of general formula (III)

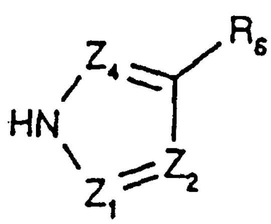

(III)

in which
$Z_1$, $Z_2$, $Z_4$ and $R_6$ have the meaning indicated above;
3.2/ simultaneous "one pot" reaction between a derivative of general formula (IV), an alkylating agent of general

formula (V) and a nitrogenous heterocycle of general formula (III)

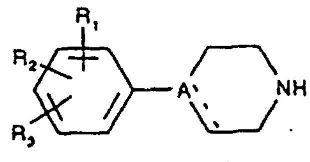

(IV)

$X-(CH_2)n-X$

(V)

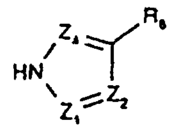

(III)

where
$R_1$, $R_2$, $R_3$, A, X, n, $Z_1$, $Z_2$, $Z_4$ and $R_6$ have the meaning indicated above;
<u>3.3</u>/ reaction under alkylation conditions of amines of general formula (IV)

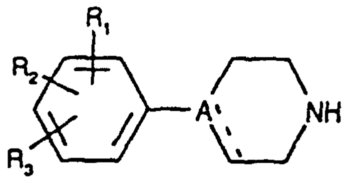

(IV)

in which
$R_1$, $R_2$, $R_3$ and A have the meaning indicated above,
with the derivatives of general formula (VI)

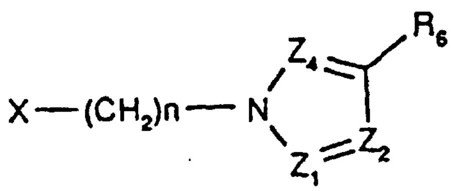

(VI)

in which
X, n, $Z_1$, $Z_2$, $Z_4$ and $R_6$ have the meaning indicated above;
<u>3.4</u>/ reaction under alkylation conditions of a compound of general formula (VII)

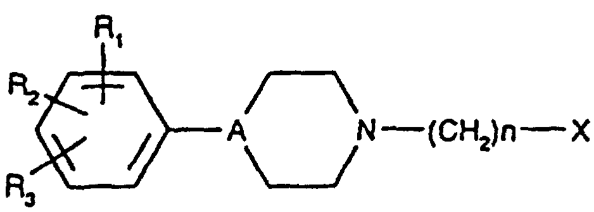

(VII)

in which
$R_1$, $R_2$, $R_3$, n, X and A have the meaning indicated above, with a nitrogenous heterocycle of general formula (III)

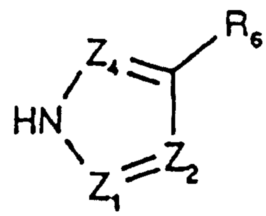

( III )

in which

$Z_1$, $Z_2$, $Z_4$ and $R_6$ have the meaning indicated above;

3.5/ dehydration of compounds of general formula (I)

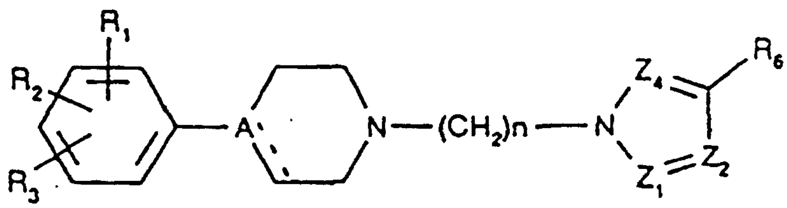

( I )

in which

$R_1$, $R_2$, $R_3$, n, $Z_1$, $Z_2$, $Z_4$ and $R_6$ have the meaning indicated above, A represents a carbon atom linked to a hydroxyl group (C-OH) and the dotted line represents the absence of an additional bond;

3.6/ addition of organometallic reagents, for example phenyllithium or phenylmagnesium bromide, to compounds of general formula (VIII)

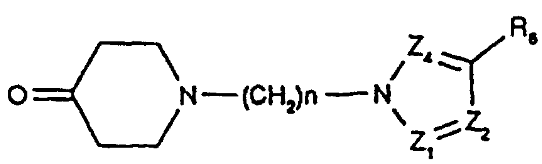

( VIII )

in which

n, $Z_1$, $Z_2$, $Z_4$ and $R_6$ have the meaning indicated above;

3.7 reduction of compounds of general formula (IX)

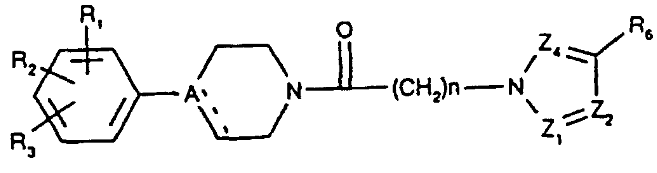

( IX )

in which
$R_1$, $R_2$, $R_3$, A, n, $Z_1$, $Z_2$, $Z_4$ and $R_6$ have the meaning indicated above.

**4.** Compounds of general formula (I) and their physiologically acceptable salts according to either of Claims 1 and 2 as medicaments, in particular as medicaments possessing therapeutic activity for the treatment of anxiety, psychosis, epilepsy, convulsion, motoricity problems, amnesia, cerebrovascular diseases or senile dementia.

**5.** Pharmaceutical compositions, **characterized in that** they contain, in addition to a pharmaceutically acceptable excipient, at least one compound of general formula (I) or one of its physiologically acceptable salts according to either of Claims 1 and 2.

**Patentansprüche**

**1.** Verbindungen der allgemeinen Formel (1)

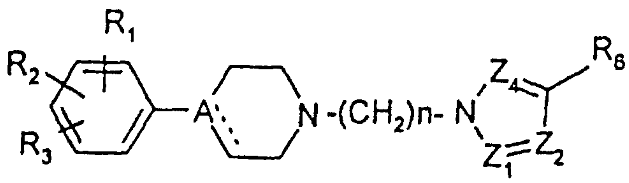

und ihre physiologisch annehmbaren Salze, in der

| | |
|---|---|
| $R_1$, $R_2$ und $R_3$, | identisch oder verschieden, jeweils ein Wasserstoffatom, ein Halogenatom, einen linearen oder verzweigten Alkylrest, der 1 bis 6 Kohlenstoffatome umfasst, einen Perfluoralkylrest, einen Phenylrest, der gegebenenfalls mit einem Halogen oder einem linearen oder verzweigten, 1 bis 6 Kohlenstoffatome umfassenden Alkylrest substituiert ist, oder einen Alkoxyrest darstellen, wobei zwei benachbarte Reste einen aromatischen oder gesättigten Zyklus bilden können; |
| A | ein Kohlenstoffatom darstellt und die gestrichelte Linie eine zusätzliche Bindung darstellt; |
| n | für 4 steht; |
| $Z_1$ | ein Stickstoffatom oder ein substituiertes Kohlenstoffatom darstellt, das durch C-$R_4$ dargestellt werden kann; |
| $Z_2$ | ein Stickstoffatom oder ein substituiertes Kohlenstoffatom darstellt, das durch C-$R_5$ dargestellt werden kann; |
| $Z_4$ | ein Stickstoffatom oder ein substituiertes Kohlenstoffatom darstellt, das durch C-$R_7$ dargestellt werden kann; |
| $R_4$, $R_5$, $R_6$ und $R_7$, | identisch oder verschieden, ein Wasserstoffatom, ein Halogenatom, einen linearen oder verzweigten Alkylrest, der 1 bis 6 Kohlenstoffatome umfasst, einen Hydroxylrest, einen Alkoxyrest, einen Phenylrest, der gegebenenfalls mit einem Halogen oder einem linearen oder verzweigten, 1 bis 6 Kohlenstoffatome umfassenden Alkylrest substituiert ist, darstellen, oder auch zwei benachbarte Reste Teil eines anderen Cyclus, aromatisch oder nicht, sein können, |

wobei die Gruppe

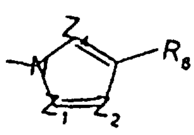

keine Carbonyl-Funktion enthält und keinen tricyclischen Rest darstellt.

**2.** Verbindungen, die in der allgemeinen Formel (1) gemäß Anspruch 1 eingeschlossen sind und aus der folgenden Gruppe ausgewählt sind:

1. 4-Chlor-1-[4-(4-hydroxy-4-phenyl-1-piperidinyl)butyl]-1H-pyrazol
2. 4,5-Dichlor-1-[4-(4-hydroxy-4-phenyl-1-piperidinyl)butyl]-2-methyl-1H-imidazol
3. 1-[4-(4-Hydroxy-4-phenyl-1-piperidinyl)butyl]-1H-benzimidazol
4. 1-[4-Hydroxy-4-phenyl-1-piperidinyl)butyl]-1H-1,2,4-triazol
5. 4-Chlor-1-{4-[4-(4-chlorphenyl)-4-hydroxy-1-piperidinyl]butyl}-1Hpyrazol
6. 4,5-Dichlor-1-{4-[4-hydroxy-4-(4-chlorphenyl)-1-piperidinyl]butyl}-2-methyl-1H-imidazol
7. 4-Chlor-1-{4-[4-hydroxy-4-(3-trifluormethylphenyl)-1-piperidinyl]butyl}-1H-pyrazol
8. 4,5-Dichlor-1-{4-[4-hydroxy-4-(3-trifluormethylphenyl)-1-piperidinyl]butyl}-2-methyl-1H-imidazol
9. 4,5-Dichlor-1-{4-[4-(4-fluorphenyl)-4-hydroxy-1-piperidinyl]butyl}-2-methyl-1H-imidazol
10. 1-[4-(4-Hydroxy-4-phenyl-1-piperidinyl)butyl}indol
11. 4,5-Dichlor-1-{4-[4-hydroxy-4-(4-methylphenyl)-1-piperidinyl]butyl}-2-methyl-1H-imidazol
12. 1-[4-(4-Hydroxy-4-phenyl-1-piperidinyl)butyl]-1H-pyrazol
13. 1-[4-(4-Hydroxy-4-phenyl-1-piperidinyl)butyl]-1H-indazol
14. 2-[4-(4-hydroxy-4-phenyl-1-piperidinyl)butyl]-2-H-indazol
15. 4-Chlor-1-{4-[4-hydroxy-4-(4-methylphenyl)-1-piperidinyl]butyl}-1H-pyrazol
16. 4-Chlor-1-{4-[4-hydroxy-4-(4-methoxyphenyl)-1-piperidinyl]butyl}-1Hpyrazol
17. 1-[4-(4-Hydroxy-4-phenyl-1-piperidinyl)butyl]-2-phenyl-1H-imidazol
18. 1-{4-[4-Hydroxy-4-(4-methylphenyl)-1-piperidinyl]butyl}-1H-benzimidazol
19. 4,5-Diphenyl-1-[4-(4-hydroxy-4-phenyl-1-piperidinyl)butyl]-1H-imidazol
20. 4-Chlor-1-{4-[4-hydroxy-4-(1-naphthyl)-1-piperidinyl]butyl}-1H-pyrazol
21. 4-Chlor-1-{4-[4-hydroxy-4-(2-naphthyl)-1-piperidinyl]butyl}-1H-pyrazol
22. 4-Chlor-1-{4-[4-phenyl-1-(1,2,3,6-tetrahydropyridinyl)]butyl}-1H-pyrazol
23. 1-{4-[4-Phenyl-1-(1,2,3,6-tetrahydropyridinyl)]butyl}-1H-benzimidazol
24. 1-{4-[4-Phenyl-1-(1,2,3,6-tetrahydropyridinyl)]butyl}-1H-1,2,4-triazol
25. 4-Chlor-1-{4-[4-(4-chlorphenyl)-1-(1,2,3,6-tetrahydropyridinyl)]butyl}-1H-pyrazol
26. 4,5-Dichlor-1-{4-[4-(4-chlorphenyl)-1-(1,2,3,6-tetrahydropyridinyl)]butyl}-2-methyl-1H-imidazol
27. 4-Chlor-1-{4-[4-(3-trifluormethylphenyl)-1-(1,2,3,6-tetrahydropyridinyl)]-butyl}-1H-pyrazol
28. 4,5-Dichlor-2-methyl-1-{4-[4-(3-trifluormethylphenyl)-1-(1,2,3,6-tetrahydropyridinyl)]butyl}-1H-imidazol
29. 4-Chlor-1-{4-[4-(4-fluorphenyl)-1-(1,2,3,6-tetrahydropyridinyl)]butyl}-1Hpyrazol
30. 4,5-Dichlor-1-(4-[4-(4-fluorphenyl)-1-(1,2,3,6-tetrahydropyridinyl)]butyl}-2-methyl-1H-imidazol
31. 4,5-Dichlor-1-{4-[4-phenyl-1-(1,2,3,6-tetrahydropyridinyl)]butyl}-2-methyl-1H-imidazol
32. 4,5-Dichlor-1-(4-[4-phenyl-1-(1,2,3,6-tetrahydropyridinyl)]butyl}-2-methyl-1H-imidazol-Hydrochlorid
33. 4,5-Dichlor-1-{4-[4-phenyl-1-(1,2,3,6-tetrahydropyridinyl)]butyl}-2-methyl-1H-imidazol-Hydrochlorid
34. 1-{4-[4-(4-Fluorphenyl)-1-(1,2,3,6-tetrahydropyridinyl)]butyl}indol
35. 1-{4-[4-Phenyl-1-(1,2,3,6-tetrahydropyridinyl)]butyl}indol
36. 4,5-Dichlor-2-methyl-1-{4-[4-(4-methylphenyl)-1-(1,2,3,6-tetrahydropyridinyl)]butyl}-1H-imidazol
37. 1-{4-[4-Phenyl-1-(1,2,3,6-tetrahydropyridinyl)]butyl}-1H-pyrazol
38. 1-{4-[4-Phenyl-1-(1,2,3,6-tetrahydropyridinyl)]butyl}-1H-indazol
39. 2-{4-[4-Phenyl-1-(1,2,3,6-tetrahydropyridinyl)]butyl}-2H-indazol
40. 4-Chlor-1-{4-[4-(4-methylphenyl)-1-(1,2,3,6-tetrahydropyridinyl)]butyl}-1 H-pyrazol
41. 4-Chlor-1-{4-[4-(4-methoxyphenyl)-1-(1,2,3,6-tetrahydropyridinyl)]-butyl}-1H-pyrazol
42. 2-Phenyl-1-{4-[4-phenyl-1-(1,2,3,6-tetrahydropyridinyl)]butyl}-1Himidazol
43. 1-{4-[4-(4-Methylphenyl)-1-(1,2,3,6-tetrahydropyridinyl)]butyl}-1H-benzimidazol
44. $R_4$, $R_5$, $R_6$ und $R_7$ sind nicht Teil eines anderen Cyclus, aromatisch oder nicht, 4,5-Diphenyl-1-{4-[4-phenyl-1-(1 ,2,3,6-tetrahydropyridinyl)]butyl}-1Himidazol
45. 4-Chlor-1-{4-[4-(1-naphthyl)-1-(1,2,3,6-tetrahydropyridinyl)]butyl}-1H-pyrazol
46. 4-Chlor-1 -{4-[4-(2-naphthyl)-1-(1,2,3,6-tetrahydropyridinyl)]butyl}-1H-pyrazol
47. 1-(4-[4-Phenyl-1-(1,2,3,6-tetrahydropyridinyl)]butyl}-1 H-benzimidazol-Hydrochlorid
48. 1-{4-[4-(4-Fluorphenyl)-1-(1,2,3,6-tetrahydropyridinyl)]butyl}-1H-benzimidazol
49. 1-{4-[4-(4-Fluorphenyl)-1-(1,2,3,6-tetrahydropyridinyl)]butyl}-1H-benzimidazol-Hydrochlorid
50. 4,5-Dichlor-2-methyl-1-{4-[4-(3-trifluormethylphenyl)]-1-(1,2,3,6-tetrahydropyridinyl)]butyl}-1H-imidazol-Hydrochlorid
51. 4-Chlor-1-{4-[4-(4-Fluorphenyl)-1-(1,2,3,6-tetrahydropyridinyl)]butyl}-1H-pyrazol-Hydrochlorid
52. 1-{4-[4-(4-Phenyl-1-(1,2,3,6-tetrahydropyridinyl)]butyl}-1H-indazol-Hydrochlorid
53. 4,5-Dichlor-1-{4-[4-(4-fluorphenyl)-1-(1,2,3,6-tetrahydropyridinyl)]butyl}-2-methyl-1H-imidazol-Hydrochlorid

54. 4-(4-Chlorphenyl)-1-{4-[4-phenyl-1-(1,2,3,6-tetrahydropyridinyl)]butyl}-1H-pyrazol-Hydrochlorid
55. 4-(4-Chlorphenyl)-1-{4-[4-phenyl-1-(1,2,3,6-tetrahydropyridinyl)]butyl}-1H-pyrazol
56. 1-{4-[4-(4-Fluorphenyl)-1-(1,2,3,6-tetrahydropyridinyl)]butyl}-1H-triazol-Hydrochlorid
57. 1-{4-[4-(4-Fluorphenyl)-1-(1,2,3,6-tetrahydropyridinyl)]butyl}-1H-triazol
58. 1-{4-[4-(4-Fluorphenyl)-1-(1,2,3,6-tetrahydropyridinyl)]butyl}-2-methyl-1H-benzimidazol
59. 1-{4-[4-(4-Fluorphenyl)-1-(1,2,3,6-tetrahydropyridinyl)]butyl}-1H-indazol
60. 2-{4-[4-(4-Fluorphenyl)-1-(1,2,3,6-tetrahydropyridinyl)]butyl}-2H-indazol
61. 2-{4-[4-(4-Fluorphenyl)-1-(1,2,3,6-tetrahydropyridinyl)]butyl}-2H-benzotriazol-Hydrochlorid
62. 2-{4-[4-(4-Fluorphenyl)-1-(1,2,3,6-tetrahydropyridinyl)]butyl}-2Hbenzotriazol
63. 1-{4-[4-(4-Fluorphenyl)-1-(1,2,3,6-tetrahydropyridinyl)]butyl}-1Hbenzotriazol-Hydrochlorid
64. 1-{4-[4-(4-Fluorphenyl)-1-(1,2,3,6-tetrahydropyridinyl)]butyl}-1Hbenzotriazol
65. 4-Chlor-1-{4-[4-(4-chlorphenyl)-1-(1,2,3,6-tetrahydropyridinyl)]butyl}-1H-pyrazol-Hydrochlorid
66. 1-{4-[4-Phenyl-1-(1,2,3,6-tetrahydropyridinyl)]butyl}-1H-pyrazol-Hydrochlorid
67. 1-{4-[4-Phenyl-1-(1,2,3,6-tetrahydropyridinyl)]butyl}-1H-triazol-Hydrochlorid
68. 2-Phenyl-1-{4-[4-phenyl-1-(1,2,3,6-tetrahydropyridinyl)]butyl}-1H-imidazol-Hydrochlorid
69. 1-{4-[4-Phenyl-1-(1,2,3,6-tetrahydropyridinyl)]butyl}-1H-pyrrol-Hydrochlorid
70 1-{4-[4-Phenyl-1-(1,2,3,6-tetrahydropyridinyl)]butyl}-1H-pyrrol
71. 4-(4-Chlorphenyl)-1-[4-(4-hydroxy-4-phenyl-1-piperidinyl)butyl]-1H-pyrazol
72. 1-{4-[4-(4-Fluorphenyl)-4-hydroxy-1-piperidinyl]butyl}-1H-benzimidazol
73. 4-Chlor-1-{4-[4-hydroxy-4-(3-trifluormethylphenyl)-1-piperidinyl]butyl}-1H-pyrazol
74. 1-{4-[4-(4-Fluorphenyl)-4-hydroxy-1-piperidinyl]butyl}-1H-indazol
75. 2-{4-[4-(4-Fluorphenyl)-4-hydroxy-1-piperidinyl]butyl}-2H-indazol
76. 2-{4-[4-(4-Fluorphenyl)-4-hydroxy-1-piperidinyl]butyl}-2H-benzotriazol
77. 1-{4-[4-(4-Fluorphenyl)-4-hydroxy-1-piperidinyl]butyl}-2H-benzotriazol
78. 3-Chlor-1-{4-[4-phenyl-1-(1,2,3,6-tetrahydropyridinyl)]butyl}-1H-indazol
79. 3-Chlor-1-{4-[4-phenyl-1-(1,2,3,6-tetrahydropyridinyl)]butyl}-1H-indazol-Hydrochlorid.

**3.** Verfahren zur Herstellung von Verbindungen der allgemeinen Formel (I) gemäß einem der Ansprüche 1 oder 2, **dadurch gekennzeichnet, dass** man mindestens eine der folgenden Reaktionen bewirkt:

3.1. Umsetzung einer Spiran-Verbindung der allgemeinen Formel (II)

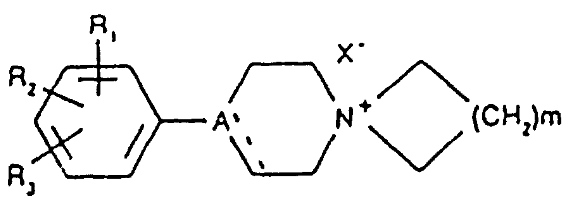

(II)

in der
$R_1$, $R_2$, $R_3$ und A die oben angegebene Bedeutung aufweisen, m für 3 steht und X eine Abgangsgruppe, wie Chlor, Brom, Mesyloxy oder Tosyloxy, darstellt,
mit einem Stickstoff-Heterocyclus der allgemeinen Formel (III)

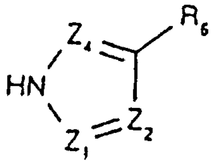

(III)

in der
$Z_1$, $Z_2$, $Z_4$ und $R_6$ die oben angegebene Bedeutung aufweisen;
3.2. gleichzeitige "Eintopf"-Reaktion zwischen einem Derivat der allgemeinen Formel (IV), einem Alkylierungsmittel der allgemeinen Formel (V) und einem Stickstoff-Heterocyclus der allgemeinen Formel (III)

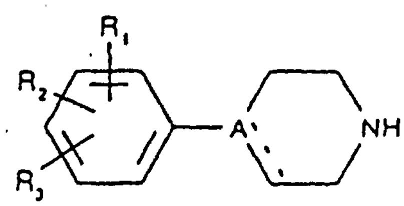

(IV)

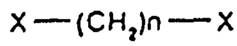

(V)

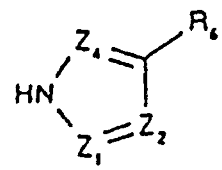

(III)

worin
$R_1$, $R_2$, $R_3$, A, X, n, $Z_1$, $Z_2$, $Z_4$ und $R_6$ die oben angegebene Bedeutung aufweisen,
3.3. Umsetzung von Aminen der allgemeinen Formel (IV)

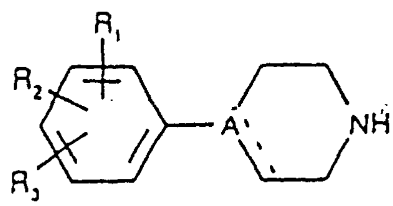

(IV)

in der
$R_1$, $R_2$, $R_3$ und A die oben angegebene Bedeutung aufweisen, mit Derivaten der allgemeinen Formel (VI)

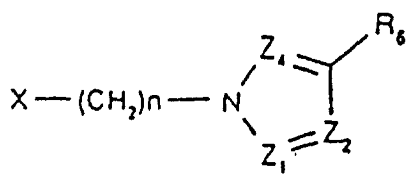

(VI)

in der
X, n, $Z_1$, $Z_2$, $Z_4$ und $R_6$ die oben angegebene Bedeutung aufweisen, unter Alkylierungsbedingungen.
3.4 Umsetzung einer Verbindung der allgemeinen Formel (VII)

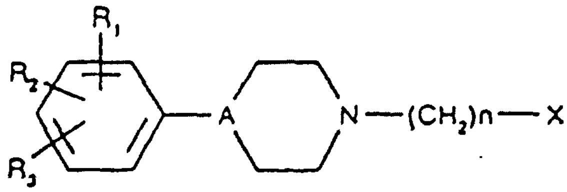

( VII )

in der
$R_1$, $R_2$, $R_3$, n, X und A die oben angegebene Bedeutung aufweisen, mit einem Stickstoff-Heterocyclus der allgemeinen Formel (III)

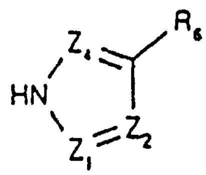

( III )

in der
$Z_1$, $Z_2$, $Z_4$ und $R_6$ die oben angegebene Bedeutung aufweisen, unter Alkylierungsbedingungen;
3.5 Dehydratisierung von Verbindungen der allgemeinen Formel (I)

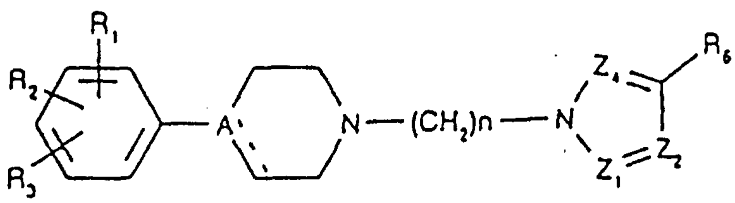

( I )

in der
$R_1$, $R_2$, $R_3$, n, $Z_1$, $Z_2$, $Z_4$ und $R_6$ die oben angegebene Bedeutung aufweisen, A ein Kohlenstoffatom darstellt, das mit einer Hydroxylgruppe vereinigt ist (C-OH), und die gestrichelte Linie das Fehlen einer zusätzlichen Bindung darstellt;
3.6 Addition von organometallischen Reagenzien - beispielsweise Phenyllithium oder Phenylmagnesiumbromid - an Verbindungen der allgemeinen Formel (VIII)

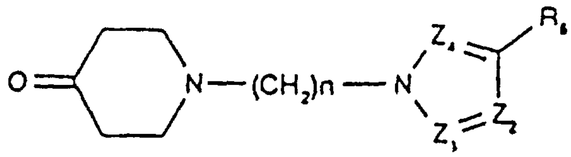

( VIII )

in der
n, $Z_1$, $Z_2$, $Z_4$ und $R_6$ die oben angegebene Bedeutung aufweisen;
3.7 Reduktion von Verbindungen der allgemeinen Formel (IX)

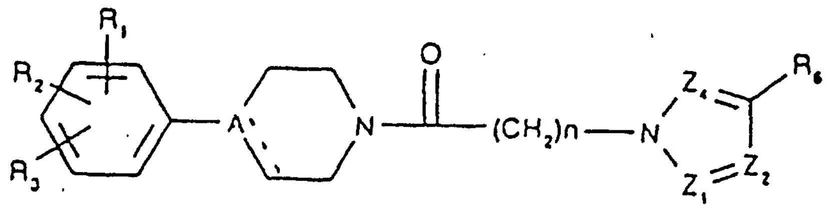

(IX)

in der
$R_1$, $R_2$, $R_3$, A, n, $Z_1$, $Z_2$, $Z_4$ und $R_6$ die oben angegebene Bedeutung aufweisen.

**4.** Verbindungen der allgemeinen Formel (I) und ihre physiologisch annehmbaren Salze gemäß einem der Ansprüche 1 oder 2 als Medikamente, insbesondere als Medikamente mit einer therapeutischen Wirkung bei der Behandlung von Angst, Psychose, Epilepsie, Krampf, Problemen der Motorik, Amnesie, zerebrovaskulären Erkrankungen, seniler Demenz.

**5.** Pharmazeutische Zusammensetzungen, **gekennzeichnet durch** die Tatsache, dass sie außer einem pharmazeutisch annehmbaren Träger mindestens eine Verbindung der allgemeinen Formel (I) oder eines ihrer physiologisch annehmbaren Salze gemäß einem der Ansprüche 1 oder 2 enthält.